# DEMANDE DE BREVET EUROPEEN

(11) **EP 0 750 042 A1**
(43) Date de publication de la demande: **27.12.1996**
(21) Numéro de dépôt: 95201669.9
(22) Date de dépôt: 20.06.1995
(51) Int. Cl.: C12N 15/52, C12N 15/74, C12N 9/00, C12N 1/21, C12P 19/14, C12Q 1/68

(54) **Bactéries lactiques produisant des exopolysaccharides**

(71) Demandeur: SOCIETE DES PRODUITS NESTLE S.A., 1800 Vevey (CH)
(72) Inventeur: Mollet, Beat, CH-1074 Mollie-Margot (CH); Stingele, Francesca, CH-1018 Lausanne (CH)

(57) **Abrégé**

Fragment d'ADN d'origine génomique codant pour au moins une enzyme impliquée dans la biosynthèse d'un EPS, et capable suite à la transformation d'une bactérie lactique de restaurer la production d'un EPS dans ladite bactérie n'en produisant pas initialement, ou de modifier la structure de l'EPS produit initialement par ladite bactérie. Protéines de la souche *Streptococcus thermophilus* CNCM I-1590 codées par le chromosome et qui sont impliquées dans la biosynthèse de l'EPS ayant la composition Glc:Gal:GalNac=1:2:1. Procédé de fabrication d'un nouvel EPS, dans lequel on clone dans un vecteur un fragment d'ADN codant partiellement ou totalement pour au moins une enzyme impliquée dans la biosynthèse d'un EPS, on transforme des bactéries lactiques produisant un autre EPS par le vecteur recombinant, puis on sélectionne une bactérie lactique produisant un nouvel EPS.

## Description

La présente invention se rapporte à l'utilisation de fragments d'ADN chromosomique de bactéries lactiques codant pour au moins une enzyme impliquée dans la biosynthèse d'exopolysaccharides, ainsi que des enzymes codées par ces fragments.

### Etat de la technique

Il est connu que les bactéries lactiques sont susceptibles de produire dans leur milieu de culture deux classes de polysaccharides, à savoir les homopolysaccharides comme les dextranes ou les levanes qui sont constitués par l'assemblage répété d'un seul sucre, et les hétéropolysaccharides appellés communément exopolysaccharides ou EPS (EPS est l'abréviation du terme "exopolysaccharide") constitués par l'assemblage de plusieurs sucres différents formant une unité répétitive (Cerning J., Bactéries lactiques, Vol I, de Rossart H et Luquet F. M., Lorica, 309-329, 1994).

Une bactérie lactique produisant un EPS peut confèrer un caractère filant et/ou une texture lisse et crémeuse à un lait acidifié (Cerning et al., FEMS Microbiol., 87, 113-130, 19/90). Les EPS peuvent aussi présenter des activités biologiques particulièrement intéressantes pour la santé humaine ou animale, comme des activités anti-tumeurs ou probiotiques, par exemple (Oda M. et al., Agric. Biol. Chem., 47, 1623-1625, 1983; EP94870139.6)

Par ailleurs, l'industrie est confrontée à une instabilité génétique de la biosynthèse des EPS dans les bactéries lactiques. Ceci se traduit généralement au cours d'une fermentation par la perte de la production d'EPS par tout ou partie des bactéries lactiques (voir "Cerning J." ci-dessus). Les produits fermentés industriels sont ainsi sujets à des variations dans leur contenu en EPS, ce qui n'est pas toujours acceptable. Pour remédier à ces problèmes, l'industrie recours actuellement à l'isolation et la caractérisation périodique de ses bactéries de manière à séparer celles qui ont perdu leur caractère originel.

La biosynthèse d'EPS dans les bactéries lactiques mésophiles, c'est à dire les bactéries lactiques ayant une croissance optimale à 28-37°C, implique au moins une enzyme qui assure l'enchaînement des sucres. Aucun gène chromosomique ou plasmidique de bactéries lactiques mésophiles codant pour une telle enzyme n'a encore été identifié et séquencé, bien que l'on connaisse des plasmides impliqués dans la biosynthèse d'EPS.

WO 92/02142 révèle ainsi l'existence du plasmide pHV67 qui produit dans *Lactococcus lactis* subsp. *lactis* (mésophile) une substance capable d'augmenter la viscosité d'un lait fermenté. US5066588 décrit deux plasmides provenant d'une souche de *Streptococcus cremoris* (mésophile) capable de confèrer un caractère épaississant à un *Streptococcus lactis.* De même, Vescovo *et al.* ont mis en évidence un plasmide d'une souche *Lactobacillus casei* subsp. *casei* (mésophile) codant pour un phénotype Muc+, c'est à dire pour des fonctions liées à la production d'épaississants exocellulaires (Vescovo *et al*., Biotechnology Letters, Vol II,709-712, 1989).

Enfin, Van den Berg *et al*. cherchent à isoler d'un *Lactobacillus sake* (mésophile) un groupe de gènes chromosomiques impliqués dans la biosynthèse d'un EPS (Van den Berg D.J.C*. et al*., First International Conference on Polysaccharide Engineering, Trondheim, Norway, June 6-8, 1994). Cependant aucun gène n'a encore été identifié et/ou séquencé.

D'un autre coté, la biosynthèse d'EPS dans les bactéries lactiques thermophiles, c'est à dire les bactéries lactiques ayant une croissance optimale à 37-45°C, n'est pas encore bien connue. On sait cependant qu'elle n'est pas associée à un plasmide. Vescovo *et al*. ont ainsi montré que le phénotype Muc+ de la souche *Lactobacillus delbrueckii* subs*p bulgaricus* 2o1 (thermophile) est lié à des fonctions chromosomiques (Vescoso et al., Biotechnology Letters, Vol II, 709-712, 1989).

Ainsi à ce jour, aucun gène ou groupe de gènes chromosomiques ou plasmidiques codant pour un EPS de bactéries lactiques mésophiles ou thermophiles n'a été identifié et/ou séquencé.

Il serait donc très intéressant d'avoir des moyens pour restaurer ou stabiliser la production originelle d'EPS dans les bactéries lactiques. De plus, il serait également intéressant d'avoir des moyens pour modifier la structure d'un EPS, et créer de ce fait de nouveaux EPS pouvant avoir des propriétés intéressantes.

### Résumé de l'invention

L'invention se destine à fournir des nouveaux moyens pour contrôler, modifier et/ou restaurer la synthèse d'EPS *in-vivo* et *in-vitro*.

A cet effet, la présente invention concerne tout fragment d'ADN de bactérie lactique d'origine chromosomique codant pour au moins une enzyme impliquée dans la biosynthèse d'un EPS, et capable suite à la transformation d'une bactérie lactique de restaurer la production d'un EPS dans ladite bactérie n'en produisant pas initialement, ou de modifier la structure de l'EPS produit initialement par ladite bactérie.

Un autre objet de la présente invention concerne les vecteurs recombinants comprenant un fragment d'ADN selon la présente invention.

Un autre objet de la présente invention concerne une protéine de la souche *Streptococcus thermophilus* CNCM I-1590 qui est codée par un gène chromosomique et qui est impliquée dans la biosynthèse de l'EPS ayant la structure répétée 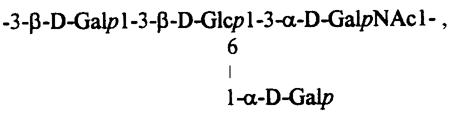 ladite protéine ayant la séquence en acides animés choisie dans le groupe formé par les séquences SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13 et SEQ ID NO:14 (séquences présentées dans la liste de séquences ci-après).

Un autre objet de la présente invention concerne une bactérie lactique comprenant, intégré dans son chromosome ou par le moyen d'un plasmide réplicable, un fragment d'ADN de bactérie lactique d'origine chromosomique codant pour au moins une enzyme impliquée dans la biosynthèse d'un EPS, ledit fragment étant capable de restaurer la production d'un EPS dans ladite bactérie n'en produisant pas initialement, ou étant capable de modifier la structure de l'EPS produit initialement par ladite bactérie.

Un autre objet de la présente invention concerne un procédé de fabrication d'un nouvel EPS, dans lequel on clone dans un vecteur un fragment d'ADN codant partiellement ou totalement pour au moins une enzyme impliquée dans la biosynthèse d'un EPS, on transforme des bactéries lactiques produisant un autre EPS par le vecteur recombinant, puis on sélectionne une bactérie lactique produisant un nouvel EPS.

La présente invention ouvre donc la possibilité d'utiliser des fragments d'ADN selon l'invention pour restaurer ou modifier la production d'EPS dans une bactérie lactique. On peut ainsi envisager d'exprimer ou de surexprimer dans une bactérie lactique l'expression des fragments selon l'invention, pour produire des EPS destinés à épaissir et rendre crémeux des boissons, des desserts liquides, des yogourts, des soupes, des crèmes glacés, des crèmes de café, des sauces ou des mayonnaises, par exemple.

La présente invention permet aussi d'avoir des moyens nouveaux pour identifier des gènes chromosomiques de bactéries lactiques impliqués dans la biosynthèse d'EPS. En effet la séquence nucléique SEQ ID NO: 1 peut servir avantageusement à la fabrication de sondes nucléiques spécifiques de gènes impliqués dans la biosynthèse d'EPS dans d'autres bactéries lactiques.

Enfin, la présente invention fournie aussi de nouvelles enzymes impliquées dans la biosynthèse de l'EPS décrit ci-dessus. Ces enzymes peuvent être ainsi avantageusement utilisées pour synthétiser ou modifier *in-vitro* un polysaccharide, comme un oligosaccharide ou un EPS, par exemple (Ichikawa Y. et al., American Chemical Society, 114, 9283-9289, 1992).

### Description détaillée de l'invention

Dans la suite de la description, le terme "EPS" désigne un exopolysaccharide produit par une bactérie lactique qui est constitué par l'assemblage de plusieurs sucres différents formant une unité répétitive.

Au sens de la présente invention, on entend par "séquence homologue" toutes séquences ne différant des séquences selon l'invention que par la substitution, la délétion ou l'addition d'un petit nombre de bases. Dans ce cadre, on considèrera en particulier comme homologue deux séquences d'ADN qui, du fait de la dégénérescence du code génétique, codent pour un même polypeptide. On considèrera aussi comme séquence homologue, celle qui présente plus de 80% d'homologie avec les séquences selon l'invention. Dans ce dernier cas, l'homologie est déterminée par le rapport entre le nombre de bases d'une séquence homologue qui sont identiques à celles d'une séquence selon l'invention, et le nombre total de bases de ladite séquence selon l'invention.

Au sens de la présente invention, on entend par "fragment qui s'hybride" tout fragment capable de s'hybrider aux fragments selon l'invention par la méthode de Southern-Blot (Sambrook et al.., Molecular Cloning, A Laboratory Manual, Cold Spring Harbor Laboratory Press, U.S.A., 1989, chapitres 9.31 à 9.58). De préférence, l'hybridation est conduite dans des conditions stringentes de manière à éviter des hybridations aspécifiques ou peu stables.

Enfin, le terme "fragment" ou "fragment d'ADN" doit être compris comme un fragment d'ADN double brin d'origine chromosomique, qui peut être synthétisé, reproduit *in-vitro* par exemple par la méthode connue appelée "Polymérase Chain Reaction", ou reproduit *in-vivo* dans une bactérie du type *Escherchia coli*, *Lactococcus lactis*, ou *Streptococcus thermophilus* par exemple.

Pour sélectionner un fragment d'ADN selon la présente invention, il est possible de constituer une banque de grands fragments d'ADN d'une bactérie lactique produisant un EPS dans une bactérie lactique ne produisant pas d'EPS, puis de sélectionner le ou les clône(s) produisant un EPS. Pour cela, on digère l'ADN génomique d'une bactérie lactique produisant un EPS par une enzyme de restriction qui est spécifique d'un site de restriction relativement rare (*Bam*HI, *Sal*I, *Pst*I) ou par une digestion partielle avec *Sau*3A, par exemple. On clone le produit de digestion dans un plasmide d'expression ou d'intégration qui accepte de grands fragments (plasmide pSA3 décrit à l'exemple II), on introduit les plasmides recombinants dans la même espèce de bactérie lactique ne produisant pas d'EPS, on sélectionne au moins un clone transformé produisant un EPS, puis on identifie, on isole et on séquence classiquement le fragment d'ADN responsable de la production d'EPS.

Vu que les fragments d'ADN selon la présente invention sont susceptibles d'être de grande taille, du fait qu'ils peuvent contenir un groupe de gènes impliqués dans la biosynthèse d'EPS, on peut préférer introduire les plasmides recombinants dans la même souche de bactérie lactique dont provienne les fragments, à la différence que cette souche a perdu la capacité de produire des EPS suite à un traitement mutagénique (traitement U.V., chimique ou par transposon).

Une alternative à la méthode décrite ci-dessus peut aussi consister à constituer une banque plasmidique de fragments d'ADN d'une souche de bactérie lactique produisant un EPS, à transformer la même souche de bactérie lactique par les plasmides incapables de s'y répliquer, à sélectionner les transformants ayant intégré un plasmide dans leur génome par recombinaison homologue (sélection par une résistance à un antibiotique, par exemple), à sélectionner les transformants ne produisant plus d'EPS, puis à isoler et séquencer les fragments d'ADN chromosomique des transformants sélectionnés qui sont adjacents au plasmide intégré. Pour cela, on peut digérer le chromosome des transformants, le liguer, puis effectuer une PCR-inverse à l'aide de sondes spécifiques du plasmide intégré ou introduire le produit de ligation dins une souche dans laquelle le plasmide recircularisé est capable de se répliquer, par exemple.

Une autre alternative à la méthode de sélection décrite ci-dessus peut aussi consister à transformer des bactéries lactiques produisant un EPS par un plasmide comprenant un transposon, à soumettre les bactéries à des conditions dans lesquelles le transposon s'excise du vecteur et s'intègre au hasard dans le génome, à sélectionner les clones de bactéries ayant perdu la capacité de produire des EPS, à isoler les fragments d'ADN génomiques desdits clones dans lesquels un transposon s'est intégré. Cette méthode est décrite plus en détail dans l'exemple I présenté ci-après.

Il faut remarquer que les méthodes de sélection décrites brièvement ci-dessus peuvent être appliquées à toutes les bactéries lactiques connues, notamment aux bactéries lactiques mésophiles comme par exemple *Streptococcus cremoris*, *Streptococcus lactis, Lactobacillus casei* subsp. *casei* et *Lactobacillus sake*, et les bactéries lactiques thermophiles comme par exemple *Streptococcus thermophilus*, *Lactobacillus delbruecki* subsp. *bulgaricus* et *Lactobacillus helveticus*. A cet effet, l'homme du métier dispose de techniques de transformation pour chaque espèce de bactérie lactique, et en particulier pour *Lactobacillus delbruecki* subsp. *bulgaricus* (Sasaki Y. et *al*., FEMS Microbiology Reviews, 12, Fourth Symposium on Lactic Acid Bacteria, Noodwijkerhout, The Netherlands, Sept 1993).

De plus, les méthodes de sélection décrites ci-dessus permettent le plus souvent d'isoler seulement une partie d'un gène ou d'un groupe de gènes impliqués dans la biosynthèse d'un EPS. Néanmoins, l'homme du métier peut facilement identifier la partie restante du gène ou du groupe de gènes en sélectionnant dans une banque chromosomique, à l'aide de sondes nucléiques basées sur un fragment isolé, un ou plusieurs clones renfermant la partie restante, par exemple (voir l'exemple I.6)

On a pu ainsi isoler un fragment d'ADN de 13.8 kb de la souche *Streptococcus thermophilus* déposé le 7 juin 1995, auprès de la Collection Nationale de Culture de Microorganisme (C.N.C.M.), Institut Pasteur, 28 rue du Dr Roux, 75724 Paris cedex 15, France, où elle a reçu le numéro de dépôt CNCM I-1590. Par ailleurs, cette souche Gram-positif présente au microscope un aspect de coques non flagellées formant des chaînettes. Cette souche ne fait pas de spores et elle est anaéorobe facultative.

Le fragment de 13.8 kb code pour des enzymes nouvelles impliquées dans la biosynthèse d'un EPS ayant la structure répétée

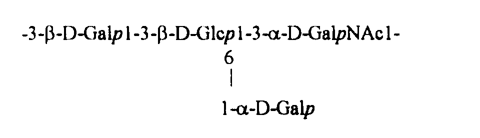

Ce fragment de 13.8 kb présente la séquence SEQ ID NO: 1 donnée dans la liste de séquence ci-après, et comporte un groupe de 13 gènes complets qui sont délimités dans la séquence nucléique SEQ ID NO: 1 par les nucléotides 8-1009, 1013-1741, 1753-2442, 2455-3201, 3257-3937, 4103-5059, 5594-6745, 6941-7417, 7426-8397, 8490-9569, 9597-10544, 10507-11427, et 11438-12733.

On a pu montrer que tout ou partie du fragment de 13.8 kb permet, suite à une transformation, de restaurer une biosynthèse d'EPS dans une bactérie lactique mésophile ou thermophile qui initialement n'en produisait pas, notamment dans un *Streptococcus*. Pour cela, on clone tout ou partie de ce fragment comprenant au moins un des gènes précités dans un plasmide d'expression en aval d'un promoteur et en amont d'un terminateur, puis on transforme un *Streptococcus* par le plasmide recombinant. En particulier, on utilise de cette façon les gènes dudit fragment pour restaurer la production d'EPS dans un mutant de la souche *S. thermophilus* CNCM I-1590 n'en produisant plus (mutant naturel ou issu d'une mutagenèse).

De même, on a pu montrer que tout ou partie du fragment de 13.8 kb peut aussi permettre, suite à une transformation, de modifier la structure répétée d'un EPS produit initialement par une bactérie lactique mésophile ou thermophile, notamment un *Streptococcus*. Ces observations ouvrent ainsi la possibilité de réaliser une méthode originale de fabrication d'un nouvel EPS, dans laquelle on clone dans un vecteur un fragment d'ADN codant partiellement ou totalement pour au moins une enzyme impliquée dans la biosynthèse d'un EPS, on transforme des bactéries lactiques produisant un autre EPS par le vecteur recombinant, puis on sélectionne une bactérie lactique produisant un nouvel EPS.

En particulier, on clone dans un vecteur d'intégration un fragment d'ADN codant partiellement pour au moins une enzyme impliquée dans la biosynthèse d'un premier EPS, on introduit le vecteur recombinant dans des bactéries lactiques mésophiles ou thermophiles produisant un deuxième EPS par l'intermédiaire d'un ou plusieurs gènes chromosomiques ou plasmidiques, on isole les bactéries ayant intégrés dans leur chromosome le vecteur d'intégration, puis on sélectionne celles qui produisent un nouvel EPS à cause de l'inactivation d'un ou plusieurs gènes impliqués dans la biosynthèse du deuxième EPS. De préférence, le premier et le deuxième EPS sont identiques, et on choisit un fragment d'ADN codant partiellement (au moins 15 paires de bases) pour au moins une enzyme impliquée dans l'adjonction d'un sucre sur la chaine latérale de l'unité répétitive ou dans la modification d'un sucre comme une sulpho-, phosphoryl- ou acétyl-transférase, par exemple.

De même, on peut cloner dans un vecteur d'expression réplicatif un fragment d'ADN codant totalement pour au moins une enzyme impliquée dans la biosynthèse d'un premier EPS, on peut introduire le vecteur recombinant dans des bactéries lactiques mésophiles ou thermophiles produisant un deuxième EPS par l'intermédiaire d'un ou plusieurs gènes chromosomiques ou plasmidiques, on peut isoler les bactéries renfermant le vecteur réplicatif, puis on peut sélectionner celles qui produisent un nouvel EPS à cause de l'expression d'un ou plusieurs gènes impliqués dans la biosynthèse du premier EPS. De préférence, on choisit des fragments d'ADN codant pour des enzymes impliquées dans la modification d'un sucre comme une sulpho-, phosphoryl- ou acétyl-transférase par exemple, ou dans l'adjonction à l'unité répétitive d'un sucre comme une glucosyl- ou une galactosyl-transférase, par exemple.

De préférence, on utilise totalement ou partiellement au moins un des gènes portés par le fragment de 13.8 kb. On peut aussi utiliser au moins un gène plasmidique de bactéries lactiques mésophiles impliqué dans la biosynthèse d'un EPS (gène que l'on peut séquencer des plasmides connus).

Enfin, le vecteur recombinant peut être tout fragment d'ADN, simple ou double brin, linéaire ou circulaire, d'expression ou d'intégration, et comprenant un fragment d'ADN selon l'invention notamment tout ou partie du fragment de 13.8 kb. Il faut toutefois veiller à ce que le fragment puisse être exprimé dans la bactérie lactique par des séquences nucléiques adaptées (promoteur; site d'attachement du ribosome; codon préféré), et le cas échéant à ce qu'il comprenne une ou plusieurs origines de réplication de diverses bactéries, notamment d'*Escherichia coli* et/ou d'un *Streptococcus*, par exemple.

L'invention concerne aussi les nouvelles enzymes codées par les gènes du fragment de 13.8 kb. On peut ainsi envisager de les utiliser pour modifier ou synthétiser *in-vitro* un oligosaccharide ou un polysaccharide comme un EPS, par exemple. Pour cela, il est préférable de purifier au moins une de ces enzymes, en surexprimant classiquement leur gène dans une bactérie et en les isolant classiquement par chromatographie, par exemple.

Un autre objet de la présente invention concerne une bactérie lactique comprenant, intégré dans son chromosome ou par le moyen d'un plasmide réplicable, un fragment d'ADN selon l'invention. De préférence, le fragment comprend au moins un des gènes du fragment de 13.8 kb.

L'invention concerne aussi toute utilisation de fragments de la séquence SEQ ID NO: 1 ou de fragments du brin complémentaire de cette séquence, d'au moins 15 paires de bases, comme amorce pour faire une PCR ou comme sonde pour détecter *in-vitro* ou inactiver *in-vivo* des gènes de bactéries lactiques impliqués dans la biosynthèse d'un EPS. Cette limite inférieure est arbitrairement fixée du fait que les petits fragments s'hybridant spécifiquement ont généralement une longeur de 15-25 pb.

La présente invention est décrite plus en détail ci-après à l'aide du complément de description qui va suivre, qui se réfère à des exemples d'obtention de fragments d'ADN, de plasmides recombinants et de bactéries transformées selon l'invention. Ces exemples sont précédés d'une description des milieux de culture. Il va de soi, toutefois, que ces exemples sont donnés à titre d'illustration de l'objet de l'invention dont ils ne constituent en aucune manière une limitation. La manipulation de l'ADN, le clonage et la transformation de cellules bactériennes sont, en l'absence de précisions contraires, effectués selon les protocoles décrits dans l'ouvrage de Sambrook et *al*. cité plus haut. Les pourcentages sont donnés en poids, sauf indication contraire.

### Milieux: (rajouter 1,5% de Bacto-agar pour un milieu solide)

- M17 (Difco,USA): tryptone 0,5%, soytone 0,5%, viande hydrolysée 0,5%, extrait de levure 0,25%, acide ascorbique 0,05%, sulphate de magnésium 0,025%, disodium-beta-glycérophosphate 1,9% et de l'eau.
- LM17: milieu M17 comprenant 1% de lactose.
- MSK: lait écrémé (poudre reconstituée à 10%) comprenant 0,1% d'extrait de levure.
- HJL; tryptone 3%, extrait de boeuf 0,2%, extrait de levure 1%, lactose 1% et KH₂PO₄ pH 6,5 0,5%.
- Rouge de Ruthénium: extrait de levure 0,5%, lait en poudre écrémé 10%, sucrose 1%, agar 1,5% et 0,08g/l de rouge de ruthénium (voir FR2632968).

### Exemple I: clonage d'un fragment d'ADN de la souche S. thermophilus Sfi6

I .1. Sélection d'une souche *S. thermophilus* productrice d'EPS : on cultive les souches de bactéries lactiques de la collection Nestlé dans un milieu liquide HJL et on en étale des dilutions sur un milieu solide Rouge de Ruthénium. Les souches productrices d'EPS demeurent de couleur blanche car les EPS empêchent le colorant de teinter leur paroi cellulaire. Par contre, les souches non-productrices se colorent en rouge du fait de l'affinité du colorant pour le peptidoglycane de leur paroi cellulaire.

On a ainsi sélectionné parmi les bactéries lactiques productrices d'EPS la souche *S*. *thermophilus* Sfi6, qui a reçu le numéro de dépôt CNCM I-1590 et que l'on désignera dans la suite des exemples par l'expression "souche Sfi6".

I.2 Structure répétée de l'EPS: la structure de l'EPS produit par la souche Sfi6 a été publiée par Doco *et al*. (Carbohyd.Res., 198, 313-321, 1995). Cet EPS présente la composition Glc:Gal:GalNac=1:2:1, et l'unité tétrasaccharidique répétée:

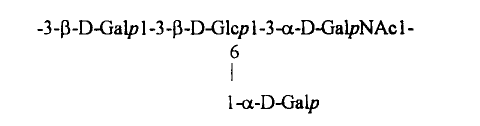

I 3. Mutagenèse par le transposon Tn*916*: on rend la souche Sfi6 résistante à la streptomycine en la cultivant par des transferts répétés dans un milieu HJL supplémenté par des teneurs croissantes de 20 à 2000µg/ml de streptomycine, puis en sélectionnant les souches devenues naturellement résistantes.

On conjugue la souche Sfi6 résistante à la streptomycine et la souche *Enterococcus faecalis* JH2-2 qui possède un plasmide pAM180 portant le transposon Tn*916* (Tn*916* est connu pour porter un gène de résistance à la tetracycline; Gawron *et al*., Nature, 300, 281-283, 1982). Pour cela, on mélange à 1ml d'une culture d'une nuit dans un milieu M17 à 37°C de la souche *E. faecalis* JH2-2, 10ml d'une culture d'une nuit dans un milieu HJL à 42°C de la souche Sfi6, on centrifuge les cellules et on les resuspend dans des tubes comprenant 100µl de milieu HJL, on dépose la suspension sur un milieu solide LM17 que l'on incube à 37°C pendant 20h, on récupère les cellules par grattage et on les resuspend dans des tubes de 10 ml de milieu liquide HJL, on incube les tubes à 42°C pendant 4h en les agitant de temps en temps, puis on étale des dilutions des cultures sur un milieu LM17 solide supplémenté de 2,5µg/ml de tetracycline et 2000µg/ml de streptomycine.

En réalisant 20 conjugaisons en parrallèles (mutations indépendantes), on a pu ainsi sélectionner 2x10⁴ transconjugants résistants à la tetracycline et à la streptomycine.

I.4 Sélection de mutants de la souche Sfi6 ne produisant plus d'EPS [phénotype EPS(-)]: on transfert les transconjugants résistants sur le milieu solide Rouge de Ruthénium supplémenté par 2,5µg/ml de tetracycline et 2000µg/ml de streptomycine. Environ 10% des transconjugants forment des colonies rouges EPS(-). On sélectionne ensuite environ 800 colonies rouges que l'on cultive une nuit dans des plaques de microtitration comprenant 200µl de milieu HJL supplémenté de 2,5µg/ml de tetracycline. On cultive ensuite 100µl de la culture HJL dans 1ml d'un lait MSK. Environ, 25% des colonies rouges testées présentent un phénotype EPS(-) stable dans le lait (le lait n'est pas épais et filant, et l'analyse du surnageant de culture ne révèle pas d'EPS). Les autres colonies rouges présentent un phénotype EPS(+) ou retrouvent le phénotype EPS(+) après plusieurs sous-cultures dans le lait.

En conclusion, les mutants stables EPS(-) ont perdu leur capacité à produire des EPS à cause de l'intégration du transposon Tn*916* dans un gène chromosomique impliqué dans la biosynthèse des EPS. En effet, les mutants stables EPS(-) peuvent retrouver un phénotype EPS(+) lorsqu'on les cultive dans un milieu de croissance dépourvu de tetracycline (excision et perte du transposon).

I.5 Caractérisation de mutants stables EPS(-): on analyse environ 100 mutants stables par Southern-blot d'une préparation d'ADN chromosomique des mutants, digérée par *Hind*III, et hybridation du filtre de Southern-blot avec le gène *tetM* radioactif (code une résistance à la tetracycline) provenant du plasmide pIC182 (Hill et al., Applied and Env. Micro., 54, 1230-1236, 1988). Environ 85% des mutants analysés présentent une bande majoritaire identique correpondant à un locus appellé "locusA". On peut remarquer pour certains des autres mutants deux autres bandes majoritaires (locus B et C) correspondant à des locus connus impliqués dans la biosynthèse de la paroi cellulaire (publication en préparation).

I.6 Caractérisation du locus A: les régions chromosomiques proches du transposon Tn*91*6 intégré peuvent être isolées par une PCR-inverse. Pour cela, on digère classiquement 1µg d'une préparation d'ADN chromosomique d'un mutant choisi arbitrairement (mutant n°1) par *Hind*III pendant 4h, on extrait l'ADN au phénol/chloroforme, on le dilue dans 720µl d'eau, on chauffe l'ADN dilué à 56°C pendant 5 min, on refroidit l'ADN sur de la glace, on lui ajoute 80µl d'un tampon de ligation 10 fois concentré et 5 unités d'une T4-ligase (Boehringer-Manheim), on l'incube à 12°C pendant 16 h, on le chauffe à 70°C pendant 15 min pour inactiver la ligase, puis on le concentre dans un volume de 100µl par plusieurs extractions successives dans du butanol. On ajoute alors dans un dispositif de PCR 10µl du mélange de ligation, 100pmol d'amorces, 15mM de dNTPs, 10µl de tampon et 0,2 unité de Super-Taq polymerase (Stehlin GmBH). Les amorces nucléiques (ou primers) sont choisies à partir de la séquence connue du transposon Tn*916*.

En utilisant les amorces ayant la séquence SEQ ID NO:15 et SEQ ID NO:16 on a pu isoler par PCR un fragment de 1kb. De plus, en utilisant les amorces SEQ ID NO:17 et SEQ ID NO:18 on a pu isoler un fragment de 4kb (voir la liste de séquences ci-après).

Un troisième fragment de 0.8kb peut être aussi isolé du mutant n°1, en réalisant une seconde PCR-inverse à partir de son ADN chromosomique digéré par R*sa*I et à l'aide des amorces ayant la séquence SEQ ID NO:18 et SEQ ID NO:19 (voir la liste de séquence ci-après).

Les fragments de 1kb et de 0.8kb ont été clonés dans le plasmide linéarisé pGEMT (Promega, USA). Le séquencage de ces fragments par la méthode des didéoxynucléotides (kit f-mol® DNA Sequencing System, Promega) montre deux séquences qui, en se recoupant, couvrent trois cadres de lectures ouvertes (ORFs) correpondants aux nucléotides 9138 à 10848 de la séquence SEQ ID NO:1.

Les fragments de 1kb et 4kb ont également été utilisés pour cribler une banque λ-ZAP Express (Stratagene, USA) renfermant des fragments d'ADN de la souche Sfi6. Pour cela, selon les recommandations du fournisseur on digère partiellement une préparation d'ADN dudit mutant par *Sau*3A, on sépare les fragments par une électrophorèse sur gel d'agarose, on coupe du gel les bandes correspondantes à des fragments de 5 à 12kb, on élue l'ADN, puis on le ligue au vecteur λ-ZAP Express préalablement digéré par *Bam*HI. On encapside *in-vitro* le produit de ligation à l'aide du système GigagoldIII (Stratagene), on mélange ensuite les phages avec des *Escherichia coli* XL1Blue (Stratagene) selon les recommandations du fournisseur, puis on étale le mélange sur boîte de Petri. On analyse ensuite les plaques recombinantes par hybridation de leur ADN transféré sur une membrane Hybond-N (Amersham Life Sciences, UK) avec les fragments de 1kb et 4kb préalablement rendus radioactifs (kit Random Primed DNA Labeling, Boehringer-Manheim).

Parmi 3000 plaques recombinantes, on a pu sélectionner par hybridation environ 20 plaques positives, desquelles on a ensuite isolé les vecteurs λ-ZAP Express, puis excisé les vecteurs pCMV renfermant un insert chromosomique (voir les recommandations du fournisseur Stratagene). Ces vecteurs recombinants sont appelés dans la suite des exemples "pFS".

On a ensuite séquencé les inserts chromosomiques de 9 vecteurs pFS (kit f-mol® DNA Sequencing System), à savoir les vecteurs pFS14, pFS26, pFS30, pFS33, pFS49, pFS50, pFS65, pFS73 et pFS80 qui comprennent respectivement des fragments correpondant aux nucléotides de la séquence SEQ ID NO: 1 8519-13807, 7193-10458, 907-7196, 566-6434, 3605-7682, 1-6881, 5202-10458, 7679-12694, et 2755-6434.

En recoupant les séquences nucléiques des différents fragments chromosomiques, on a pu ainsi déterminer la séquence SEQ ID NO:1 de 13.8kb correspondant au locus A de la souche Sfi6.

### I.7. Analyse de la séquence SEQ ID NO:1:

La séquence SEQ ID NO:1 comprend la quasi-totalité de l'opéron *eps* de la souche Sfi6. Cette séquence comprend 13 ORFs complets, dans la même orientation, que l'on appelle *epsR, A, B, C, D, E, F G, H, I, J K, L.* Cette séquence comprend en outre 1 ORF complet à l'extrémité 3' de la séquence, qui est codé par le brin complémentaire. Cet ORF, appellé *orf*Z, marque probablement la fin de l'opéron du fait de son orientation inverse par rapport aux autres ORFs.

On a ensuite comparé les séquences en acides aminés codées par les 13 premiers ORFs avec celles de protéines présentes dans la banque de donnée Swiss-Prot, à l'aide des logiciels FASTA, PEPPLOT et PILEUP de GCG-softwear, Wisconsin, USA. Les résultats sont présentés ci-après.

L'ORF incomplet *epsR* (nucléotides 8-1009) code pour une protéine EpsR (SEQ ID NO:2) ayant 26,4% d'identité avec la protéine LytR de *Bacillus subtilis* qui est impliquée dans la régulation de l'autolysine N-acetylmuramoyl-L-alanine (Lazaveric *et al*., J. Gen. Microbiol., 138, 1949-1961, 1992). EpsR est donc probablement une protéine de régulation de l'opéron *eps*. Par ailleurs, puisqu'un ORF de régulation d'un opéron est généralement trouvé en amont des autres ORFs, le gène *eps*R est probablement le premier gène de l'opéron *eps*.

Le gène *ep*s*A* (nucléotides 1013-1741) code pour une protéine EpsA (SEQ ID NO:3) ayant 67,5% d'identité avec la protéine CpsA de *Streptococcus agalactiae* et 30% d'identité avec la protéine CapC de *Staphylococcus aureus* (Rubens *et al*., Mol. Microbil., 8, 843-885, 1993; Lin et al., J. Bacteriol., 176, 7005-7016, 1994). La fonction précise de ces gènes est encore inconnue, en dehors du fait qu'ils sont essentiels pour la synthèse de la capsule qui est constituée de polysaccharides accrochés aux phospholipides de la membrane externe des bactéries.

Le gène *ep*s*B* (nucléotides 1753-2442) code pour une protéine EpsB (SEQ ID NO:4) ayant 52% d'identité avec la protéine CpsB de *Streptococcus agalactiae* qui est impliquée dans la synthèse de la capsule (Rubens *et al*.). EpsB a aussi 23% d'identité, 49% de similarité, et un profil d'hydrophobicité comparable à celui des protéines CLD de *Salmonella typhimurium, Salmonella enterica* et *Escherichia coli* (Batchelor et al., J. Bacteriol., 174, 5228-5236, 1992; Bastin et al., Mol. Microbiol., 7, 725-734, 1993). Il faut remarquer que les protéines CLD sont impliquées dans le contrôle de la longeur des chaînes de polysaccharides lors de leur biosynthèse.

Le gène *epsC* (nucléotides 2455-3201) code pour une protéine EpsC (SEQ ID NO:5) ayant 60,5% d'identité avec la protéine CpsC d*e Streptococcus agalactia*e, ayant 34,5% d'identité avec la protéine CapA de *Staphylococcus aureus*, et ayant 33% d'identité avec la protéine ExoP de *Rhizobium meliloti* (Rubens *et al*.; Lin *et al*.; Becker *et al*., Mol. Gen. Genet., 241, 367-379, 1993). La protéine ExoP est une protéine de membrane qui est impliquée dans la translocation d'EPS et/ou de précurseurs d'EPS.

Le gène *eps*D (nucléotides 3257-3937) code pour se protéine EpsD (SEQ ID NO:6) présentant des homologies significatives avec de nombreuses protéines ayant une activité galactosyl-transférase (Rubens *et al*.). Ce gène code donc probablement pour une galactosyl-transférase.

On peut remarquer que les gènes *epsA, B, C, D* de *S. thermophilus* Sfi6 sont similaires à ceux de l'opéron de *S*. *agalactiae* comprenant les gènes *cpsA, B, C, D* (Rubens *et al*.). De plus, ils sont organisés de la même façon. Bien que les polysaccharides de capsule et l'EPS des deux souches soient très différents, ceci indique qu'une région chromosomique a été probablement tranférée entre ces deux espèces.

Le gène *epsE* (nucléotides 4103-5059) code pour une protéine EpsE (SEQ ID NO:7) ayant 22% d'identité avec une protéine, codée par un ORF du cluster *rfb* de *Salmonella typhimurium*, qui est impliquée probablement en tant que glycosyl-transférase dans la biosynthèse du polysaccharide appellé "antigene O" (Jiang *et al*., Mol. Microbiol., 5, 695, 713, 1991). EpsE a aussi respectivement 24,5% et 23% d'identité avec les protéines CapH et CapM de *S. mutans* qui sont impliquées probablement en tant que glycosyl-transférases dans la biosynthèse de la capsule (Lin *et al*. ).

Le gène *epsF* (nucléotides 5594-6745) code pour une protéine EpsF (SEQ ID NO:8) ayant 20,5% d'identité et 50% de similarité avec la N-acétylglucoseamine-transférase de *Salmonella typhimurium* LT2 qui est impliquée dans la biosynthèse du polysaccharide LPS de la membrane externe (Mac Lachlan *et al*., J. Bacterioal., 173, 7151-7163, 1991). Du fait qu'une N-acétylglucosamine n'est pas impliquée dans la biosynthèse de l'EPS de la souche Sfi6 (il n'y a pas de glucose acetylé), le gène *eps*F code probablement pour une glucosyl-transférase, une N-acétylgalactosyl-transférase, ou une N-acétylglucosyl-transférase ayant une activité N-acétylglucosamine-épimérase.

Le gène *epsG (*nucléotides 6941-7417) code pour une protéine EpsG (SEQ ID NO:9) ayant de fortes homologies avec des acétyl-transférases NodL-LacA-CysE (Downie et al., Mol. Microbiol. 3, 1649-1651, 1989). De ce fait la protéine EpsG pourrait être une acétyl-transférase impliquée dans la biosynthèse de la N-acétylgalactoseamine de l'EPS.

Le gène *epsH* (nucléotides 7426-8397) code pour une protéine EpsH (SEQ ID NO: 10) ayant 24% d'identité avec une protéine, codée par un ORF du cluster *rfb* de *Salmonella typhimurium*, qui est probablement une glycosyl-transférase (Jiang *et al*.).

Le gène *epsI* (nucléotides 8490-9569) code pour une protéine EpsI (SEQ ID NO: 11) ayant 20% d'identité et un profil d'hydrophobicité comparable à celui d'une protéine d'un ORF du cluster *rfb* de *Salmonella enterica* qui est lui-même similaire à une polymérase de l'antigène O des salmonelles du groupe B et C2 (Lee et al., J. Gen, Microbiol., 138, 1843-1855, 1992; Morona et al., J. Bacteriol. 176, 733-747, 1994). Le gène *epsI* pourrait donc coder une EPS-polymérase qui polymériserait l'unité tétrasaccharide de l'EPS.

Le gène *epsJ* (nucléotides 9597-10544) code pour une protéine EpsJ (SEQ ID NO: 12) ayant 18% d'identité et 42% de similarité avec la protéine, codée par le gène *lip*B de *Neisseria meningitidis*, qui est impliquée dans la biosynthèse de la capsule en accrochant des polysaccharides aux phospholipides de la membrane externe (Frosch *et al*., Mol. Microbiol., 8, 483-493, 1993). Sachant que les *S*. *thermophilus* n'ont pas de membrane externe (Gram-positif, le gène *epsJ* pourrait donc coder une enzyme impliquée dans l'accrochage des EPS aux phospholipides de la membrane cellulaire, qui de concert avec un transporteur d'EPS (probablement EpsC) et une enzyme qui détache les EPS, participerait au transport de l'EPS à travers la membrane (modèle en accord avec celui présenté par Frosch *et al*. ).

Par ailleurs, on peut remarquer que le transposon Tn*916* est intégré dans le gène *epsJ* du mutant n°1 utilisé pour identifier l'opéron *eps* (voir le point I.6 ci-dessus), entre les nucléotides 9745-9746 de la séquence SEQ ID NO:1.

Le gène *epsK* (nucléotides 10507-11427) code pour une protéine EpsK (SEQ ID NO: 14) qui ne présente aucune homologie avec des protéines connues. Les 39 premiers nucléotides sont couverts par l'extrémité 3' de *epsJ*, ce qui laisse supposer une expression coordonnée des deux protéines, et une activité de la protéine EpsK dans le transport membranaire de l'EPS.

Le gène *epsL* (nucléotides 11438-12733) code pour une protéine EpsL (SEQ ID NO: 13) qui ne présente aucune homologie avec des protéines connues de la banque de données Swiss-prot. Ce gène est certainement impliqué dans la biosynthèse de l'EPS de la souche Sfi6 car il n'y a pas, en amont, un promoteur spécifique pour ce gène.

Le gène *orf*Z (12935-13510 sur le brin complémentaire) est présent en orientation inverse par rapport au reste des ORFs de l'opéron *eps*. De ce fait, il n'est probablement pas impliqué dans la biosynthèse de l'EPS de la souche Sfi6. De plus, il ne présente aucune homologie avec des protéines connues de la banque de données Swiss-prot.

En conclusion, les inserts chromosomiques isolés des 9 vecteurs pSF (voir le point I.6 ci-dessus) couvrent une région chromosomique de la souche *S. thermophilus* Sfi6 qui est manifestement impliquée dans la biosynthèse de l'EPS. On a pu ainsi identifier 13 gènes complets qui comprennent en amont un promoteur délimitant le début de l'opéron *eps* (résultats non montrés)

### Exemple II: inactivation du gène epsI

On inactive par recombinaison homologue le gèn*e epsI* de l'opéron *eps* pour confirmer son importance dans la biosynthèse de l'EPS.

Pour cela, on isole un fragment *Dra*I-*Sal*I du plasmide pGEMT renfermant le fragment de PCR de 0.8 kb (voir l'exemple I.6 ci-dessus), on le ligue dans le plasmide thermosensible pSA3 (Dao *et al*., Appl. Environ. Microbiol., 49, 115-119, 1985) préalablement digéré par *Eco*RV et *Sal*I, on transforme la souche *E*. *coli* XL1-blue par le produit de ligation, on sélectionne des transformants, on isole un plasmide recombinant, puis on transforme par électroporation la souche *S*. *thermophilus* Sfi6 avec le plasmide recombinant au moyen d'une méthode adaptée de celle décrite par Slos *et al*. (Appl. Environ. Microbiol., 57, 1333-1339, 1991). On resuspend les cellules soumises à une décharge de 2,1kV, 25µF et 400Ω dans 1ml de milieu HJL que l'on incube 4h à 37°C (température permissive), on étale les cellules sur un milieu solide LM17 supplémenté de 2,5µg/ml d'erythromycine que l'on incube 16h à 37°C, puis on sélectionne les colonies transformées qui survivent. On incube ensuite les colonies sélectionnées dans 2 ml de milieu HJL supplémenté de 2,5µg/ml d'erythromycin jusqu'à ce que la densité optique à 600nm (DO₆₀₀) de la culture atteigne 0,2, on soumet la culture à 45°C jusqu'à ce que la DO₆₀₀ atteigne 1.0 (le plasmide ne se réplique plus), puis on étale des dilutions de la culture sur un milieu LM17 solide supplémenté de 2,5µg/ml d'erythromycine que l'on incube 12h à 45°C.

Les colonies qui survivent ont intégré dans le gène *epsI* le plasmide pSA3 recombinant. Ceci peut être vérifié par Southern-Blot d'une préparation d'ADN chromosomique des colonies survivantes digérée par *Eco*RI (coupe une seule fois dans pSA3), et hybridation du filtre de Southern-Blot avec le fragment radioactif précité *Dra*I-*Sal*I. Les colonies ayant intégré le plasmide pSA3 présentent deux bandes sur le filtre de Southern-Blot. De plus, les colonies ayant intégré dans *eps*I le plasmide pSA3 recombinant présentent un phénotype EPS(-) sur un milieu solide Rouge de Ruthénium, et ont perdu leur caractère filant dans un lait MSK (voir l'exemple I.4 ci-dessus).

### Exemple III: inactivation des gènes epsR, A, B, C, D, E, F, G, H, K, L

On a montré aux exemples I et II que l'inactivation des gènes *epsJ* et *epsI*, par insertion d'un transposon ou d'un plasmide intégratif, interrompt la biosynthèse d'EPS dans la souche Sfi6.

De même, on peut inactiver par recombinaison homologue les autres gènes de l'opéron *eps* de la souche Sfi6, et observer ainsi une interruption de la biosynthèse d'EPS. Pour cela, on amplifie par PCR un fragment d'un ORF provenant d'un des 9 vecteurs pFS décrits à l'exemple I.6 ci-dessus. On le clone dans le plasmide pSA3, puis on le transforme et on l'intègre à la souche Sfi6 dans les mêmes conditions que celles décrites à l'exemple précédent.

### Exemple IV: restauration de la production d'EPS

On coupe par *Eco*RI pFS30, on sépare les fragments, on ligue le fragment de 5.5 kb à pFS14 préalablement digéré par *Eco*RI, on transforme des cellules XL1-blue par le produit de ligation, on sélectionne des clones transformés présentant une bonne orientation des inserts, on isole un plasmide appellé pFS30-14, on ligue un fragment *Eco*RI central de pFS65 à pFS30-14 préalablement coupé par *Eco*RI, on transforme des cellules XL1-blue par le produit de ligation, puis on sélectionne des clones transformés présentant une bonne orientation des inserts. Le plasmide recombinant résultant, appellé pFS30-65-14, comprend les nucléotides 907 à 13807 de la séquence SEQ ID NO:1.

On coupe ensuite pFS30-65-14 par *Sal*I et *Sma*l, on sépare le fragment de 12.9 kb, on le ligue à pSA3 préalablement coupé par *Eco*RV et *Sal*I, on transforme des cellules XL1-blue par le produit de ligation, on sélectionne des clones transformés, et on isole des plasmides pSA3 recombinants.

On transforme par électroporation la souche *S. thermophilus* CNCM I-1292 déposée le 29 mars 1993 par les plasmides pSA3 recombinants. Cette souche Gram-positive présente au microscope un aspect de coques non flagellées formant des chaînettes, elle ne fait pas de spores, elle est anaéorobe facultative, elle ne produit pas d'EPS, et elle présente dans son génome 1000 pb correspondant à l'extrémité 5' de l'operon *eps*. Le plasmide pSA3 recombinant peut donc s'intégrer dans le génome de la souche CNCM I-1292. Certains des clones transformés présentent un phénotype EPS+ sur un milieu solide Rouge de Ruthénium, et un caractère filant dans un lait MSK.

### Exemple V restauration de la production d'EPS

On coupe le chromosome de la souche Sfi6 par des enzymes qui ne coupent pas dans la séquence SEQ ID NO:1 (*Bam*HI, *Sal*I, *Nru*I, *Stu*I), on sépare le produit de digestion sur un gel d'agarose, on élue les bandes de 15-25 kb, on les ligue dans pSA3 préalablement coupé par une enzyme de restriction appropriée, on transforme par électroporation la souche *S. thermophilus* CNCM I-1292, puis on sélectionne des transformants par transferts des colonies sur un filtre suivi d'une hybridation de leur ADN avec l'insert de pFS14 rendu préalablement radioactif. Certains des clones transformés présentent un phénotype EPS+ sur un milieu solide Rouge de Ruthénium, et un caractère filant dans un lait MSK.

### Example VI modification d'un EPS

On transforme par électroporation la souche *S. thermophilus* CNCM I-1422, déposée le 18 mai 1994, par le plasmide pSA3 recombinant de l'exemple V. Cette souche Gram-positive présente au Microscope un aspect de coques non flagellées formant des chaînettes, elle ne fait pas de spores, elle est anaéorobe facultative, et elle produit un EPS ayant la composition Glc:Gal=2:2.

### Exemple VII modification d'un EPS

On transforme par électroporation la souche *S. thermophilus* CNCM I-1351, déposée le 5 août 1993, par le plasmide pSA3 recombinant de l'exemple V. Cette souche Gram-positive présente au microscope un aspect de coques non flagellées formant des chaînettes, elle ne fait pas de spores, elle est anaéorobe facultative, et elle produit un EPS ayant la composition Glc:Gal:Rha=1:3:2

## Revendications

1. Fragment d'ADN d'origine chromosomique codant pour au moins une enzyme impliquée dans la biosynthèse d'un EPS, et capable suite à la transformation d'une bactérie lactique de restaurer la production d'un EPS dans ladite bactérie n'en produisant pas initialement, ou de modifier la structure de l'EPS produit initialement par ladite bactérie.

2. Fragment d'ADN selon la revendication 1, codant pour au moins une enzyme impliquée dans la biosynthèse de l'EPS présentant la structure répétée

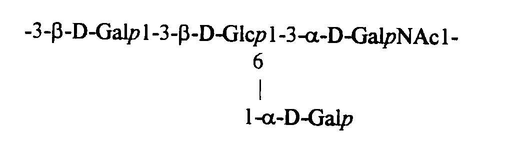

3. Fragment d'ADN selon la revendication 2, présentant la séquence nucléique SEQ ID NO:1.

4. Fragment d'ADN selon la revendication 2 comprenant au moins un gène choisi dans le groupe de gènes délimités dans la séquence nucléique SEQ ID NO:1 par les nucléotides 8-1009, 1013-1741, 1753-2442, 2455-3201, 3257-3937, 4103-5059, 5594-6745, 6941-7417, 7426-8397, 8490-9569, 9597-10544, 10507-11427, et 11438-12733.

5. Fragment d'ADN selon la revendication 2, qui est homologue ou qui s'hybride à un fragment d'ADN selon l'une des revendications 3 et 4.

6. Vecteur recombinant comprenant un fragment d'ADN selon l'une des revendications 1 à 5.

7. Protéine de la souche *Streptococcus thermophilus* CNCM I- 1590 qui est codée par un gène chromosomique et qui est impliquée dans la biosynthèse de l'EPS ayant la structure répétée 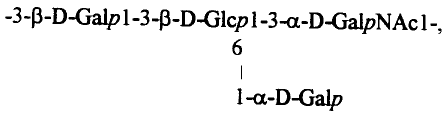 ladite protéine ayant la séquence en acides aminés choisie dans le groupe formé par les séquences SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13 et SEQ ID NO:14.

8. Bactérie lactique comprenant, intégré dans son génome ou par le moyen d'un plasmide réplicable, un fragment d'ADN chromosomique d'une bactérie lactique, ledit fragment codant pour au moins une enzyme impliquée dans la biosynthèse d'un EPS, et étant capable de restaurer la production d'un EPS dans ladite bactérie n'en produisant pas initialement, ou étant capable de modifier la structure d'un EPS produit initialement par ladite bactérie.

9. Procédé de fabrication d'un nouvel EPS, dans lequel on clone dans un vecteur un fragment d'ADN codant partiellement ou totalement pour au moins une enzyme impliquée dans la biosynthèse d'un EPS, on transforme des bactéries lactiques produisant un autre EPS par le vecteur recombinant, puis on sélectionne une bactérie lactique produisant un nouvel EPS.

10. Procédé selon la revendication 9, dans lequel on clone dans un vecteur un fragment d'ADN selon l'une des revendications 2 à 5.

11. Utilisation de fragments d'ADN de la séquence SEQ ID NO:1 ou de son brin complémentaire, d'au moins 15pb, comme amorce pour faire une PCR ou comme sonde pour détecter *in-vitro* ou inactiver *in-vivo* des gènes de bactéries lactiques impliquées dans la biosynthèse d'un EPS.


LISTE DE SEQUENCES

(1) INFORMATIONS GENERALES:
    (i) DEPOSANT:
        (A) NOM: SOCIETE DES PRODUITS NESTLE
        (B) RUE: AVENUE NESTLE 55
        (C) VILLE: VEVEY
        (D) ETAT OU PROVINCE: CANTON DE VAUD
        (E) PAYS: SUISSE
        (F) CODE POSTAL: 1800
        (G) TELEPHONE: (41) 21 924 4760
        (H) TELECOPIE: (41) 21 924 2880
    (ii) TITRE DE L' INVENTION: BACTERIES LACTIQUES PRODUISANT DES EPS
    (iii) NOMBRE DE SEQUENCES: 19
    (iv) FORME DECHIFFRABLE PAR ORDINATEUR:
        (A) TYPE DE SUPPORT: Floppy disk
        (B) ORDINATEUR: IBM PC compatible
        (C) SYSTEME D' EXPLOITATION: PC-DOS/MS-DOS
        (D) LOGICIEL: PatentIn Release #1.0, Version #1.30 (OEB)


(2) INFORMATIONS POUR LA SEQ ID NO: 1:
    (i) CARACTERISTIQUES DE LA SEQUENCE:
        (A) LONGUEUR: 13807 paires de bases
        (B) TYPE: nucléotide
        (C) NOMBRE DE BRINS: double
        (D) CONFIGURATION: linéaire
    (ii) TYPE DE MOLECULE: ADN (génomique)
    (ix) CARACTERISTIQUE:
        (A) NOM/CLE: CDS
        (B) EMPLACEMENT:8..1009
    (ix) CARACTERISTIQUE:
        (A) NOM/CLE: CDS
        (B) EMPLACEMENT:1013..1741
    (ix) CARACTERISTIQUE:
        (A) NOM/CLE: CDS
        (B) EMPLACEMENT:1753..2442
    (ix) CARACTERISTIQUE:
        (A) NOM/CLE: CDS
        (B) EMPLACEMENT:2455..3201
    (ix) CARACTERISTIQUE:
        (A) NOM/CLE: CDS
        (B) EMPLACEMENT:3257..3937
    (ix) CARACTERISTIQUE:
        (A) NOM/CLE: CDS
        (B) EMPLACEMENT:4103..5059
    (ix) CARACTERISTIQUE:
        (A) NOM/CLE: CDS
        (B) EMPLACEMENT:5594..6745
    (ix) CARACTERISTIQUE:
        (A) NOM/CLE: CDS
        (B) EMPLACEMENT:6941..7417
    (ix) CARACTERISTIQUE:
        (A) NOM/CLE: CDS
        (B) EMPLACEMENT:7426..8397
    (ix) CARACTERISTIQUE:
        (A) NOM/CLE: CDS
        (B) EMPLACEMENT:8490..9569
    (ix) CARACTERISTIQUE:
        (A) NOM/CLE: CDS
        (B) EMPLACEMENT:9597..10544
    (ix) CARACTERISTIQUE:
        (A) NOM/CLE: misc_feature
        (B) EMPLACEMENT:10507..11427
        (D) AUTRES INFORMATIONS:/product= "CDS region overlapping
            CDS 9597-10544"
    (ix) CARACTERISTIQUE:
        (A) NOM/CLE: CDS
        (B) EMPLACEMENT:11438..12733
    (ix) CARACTERISTIQUE:

(A) NOM/CLE: misc_feature
(B) EMPLACEMENT:12935..13510
(D) AUTRES INFORMATIONS:/function= "cadre de lecture
ouverte porte par le brin complementaire"

(xi) DESCRIPTION DE LA SEQUENCE: SEQ ID NO: 1:

GATCTTG ATG TCA GCT CTC AAA AAA GAT AAA AAA GTT GAT GTT AAA GTT 49
Met Ser Ala Leu Lys Lys Asp Lys Lys Val Asp Val Lys Val
1               5                   10

GAT GAT GTT GCC TCA TAT CAA GAA GCT TAT GAT AAT CTC AAG TCT GGC 97
Asp Asp Val Ala Ser Tyr Gln Glu Ala Tyr Asp Asn Leu Lys Ser Gly
15                  20                  25                  30

AAA TCT AAA GCT ATG GTC TTG AGT GGC TCT TAT GCT AGC CTA TTA GAG 145
Lys Ser Lys Ala Met Val Leu Ser Gly Ser Tyr Ala Ser Leu Leu Glu
                35                  40                  45

TCT GTC GAT AGT AAT TAT GCT TCA AAT CTA AAA ACA ATT TAT ACT TAT 193
Ser Val Asp Ser Asn Tyr Ala Ser Asn Leu Lys Thr Ile Tyr Thr Tyr
            50                  55                  60

AAA ATT AAA AAG AAG AAT AGC AAC TCT GCA AAC CAA GTA GAT TCA AGA 241
Lys Ile Lys Lys Lys Asn Ser Asn Ser Ala Asn Gln Val Asp Ser Arg
        65                  70                  75

GTC TTC AAT ATT TAT ATT AGT GGT ATT GAT ACC TAC GGT CCG ATT TCA 289
Val Phe Asn Ile Tyr Ile Ser Gly Ile Asp Thr Tyr Gly Pro Ile Ser
    80                  85                  90

ACA GTG TCA CGT TCA GAT GTC AAT ATC ATT ATG ACA GTA AAC ATG AAT 337
Thr Val Ser Arg Ser Asp Val Asn Ile Ile Met Thr Val Asn Met Asn
95                  100                 105                 110

ACA CAT AAG ATT CTC TTG ACG ACT ACT CCA CGT GAT GCA TAC GTT AAG 385
Thr His Lys Ile Leu Leu Thr Thr Thr Pro Arg Asp Ala Tyr Val Lys
                115                 120                 125

ATT CCT GGT GGT GGG GCA GAC CAG TAT GAT AAA TTA ACC CAC GCA GGT 433
Ile Pro Gly Gly Gly Ala Asp Gln Tyr Asp Lys Leu Thr His Ala Gly
            130                 135                 140

ATT TAT GGC GTT GAA ACA TCT GAA CAA ACT CTA GAA GAT CTT TAT GGT 481
Ile Tyr Gly Val Glu Thr Ser Glu Gln Thr Leu Glu Asp Leu Tyr Gly
        145                 150                 155

ATT AAG CTT GAT TAC TAT GCA CGA ATT AAC TTC ACA TCT TTC CTT AAG 529
Ile Lys Leu Asp Tyr Tyr Ala Arg Ile Asn Phe Thr Ser Phe Leu Lys
    160                 165                 170

TTG ATT GAC CAA CTT GGT GGT GTG ACA GTC CAT AAT GAT CAA GCT TTC 577
Leu Ile Asp Gln Leu Gly Gly Val Thr Val His Asn Asp Gln Ala Phe
175                 180                 185                 190

ACA CAA GAG AAG TTT GAT TTC CCG GTT GGA GAT ATC CAA ATG AAT TCA 625
Thr Gln Glu Lys Phe Asp Phe Pro Val Gly Asp Ile Gln Met Asn Ser
                195                 200                 205

GAG CAA GCA CTT GGA TTT GTT CGT GAA CGC TAT AAT TTA GAT GGC GGA 673
Glu Gln Ala Leu Gly Phe Val Arg Glu Arg Tyr Asn Leu Asp Gly Gly
            210                 215                 220

GAT AAT GAC CGT GGT AAA AAC CAG GAG AAA GTT ATT TCT GCG ATT TTA 721
Asp Asn Asp Arg Gly Lys Asn Gln Glu Lys Val Ile Ser Ala Ile Leu
        225                 230                 235

AAC AAG TTG GCT TCT CTA AAA TCT GTA TCA AAC TTT ACT TCA ATC GTT 769
Asn Lys Leu Ala Ser Leu Lys Ser Val Ser Asn Phe Thr Ser Ile Val
    240                 245                 250

AAT AAT CTC CAA GAC TCT GTC CAA ACG AAT ATG TCT TTG AAT ACC ATT 817
Asn Asn Leu Gln Asp Ser Val Gln Thr Asn Met Ser Leu Asn Thr Ile
255                 260                 265                 270

AAC GCT TTG GCT AAT ACA CAA CTT GAA TCA GGT TCT AAA TTT ACG GTG 865
Asn Ala Leu Ala Asn Thr Gln Leu Glu Ser Gly Ser Lys Phe Thr Val
                275                 280                 285

ACT TCT CAA GCA GTA ACA GGT ACA GGT TCA ACC GGA CAA TTG ATC TCT 913
Thr Ser Gln Ala Val Thr Gly Thr Gly Ser Thr Gly Gln Leu Ile Ser
            290                 295                 300

TAT GCG ATG CCA AAT TCT AGT CTT TAC ATG ATG AAA CTA GAT AAT TCG 961
Tyr Ala Met Pro Asn Ser Ser Leu Tyr Met Met Lys Leu Asp Asn Ser
        305                 310                 315

AGT GTG GAA AGT GCC TCT CAA GCT ATC AAA AAA TTG ATG GAG GAA AAA 1009
Ser Val Glu Ser Ala Ser Gln Ala Ile Lys Lys Leu Met Glu Glu Lys
    320                 325                 330

TAA GTG ATT GAC GTT CAC TCA CAT ATT GTT TTT GAT GTT GAT GAT GGT 1057
Val Ile Asp Val His Ser His Ile Val Phe Asp Val Asp Asp Gly
1 5 10 15

CCT GAA ACT TTA GAA GAA AGT TTA GAC CTC ATT GGT GAA AGT TAC GCC 1105
Pro Glu Thr Leu Glu Glu Ser Leu Asp Leu Ile Gly Glu Ser Tyr Ala
20 25 30

CAG GGG GTA CGT AAG ATT GTT TCA ACA TCC CAT CGT CGT AAG GGG ATG 1153
Gln Gly Val Arg Lys Ile Val Ser Thr Ser His Arg Arg Lys Gly Met
35 40 45

TTT GAG ACT CCA GAG GAT AAA ATT TTT GCC AAC TTT AAA AAA GTA AAA 1201
Phe Glu Thr Pro Glu Asp Lys Ile Phe Ala Asn Phe Lys Lys Val Lys
50 55 60

GCA GAA GCA GAA GCA CTT TAT CCA GAC TTA ACT ATT TAT TAT GGA GGT 1249
Ala Glu Ala Glu Ala Leu Tyr Pro Asp Leu Thr Ile Tyr Tyr Gly Gly
65 70 75

GAA CTT TAT TAC ACC TCA GAC ATT GTG GAG AAA CTT GAA AAG AAT CTC 1297
Glu Leu Tyr Tyr Thr Ser Asp Ile Val Glu Lys Leu Glu Lys Asn Leu
80 85 90 95

ATT CCG CGC ATG CAC AAC ACT CAA TTT GCT TTA ATT GAG TTT AGT GCT 1345
Ile Pro Arg Met His Asn Thr Gln Phe Ala Leu Ile Glu Phe Ser Ala
100 105 110

CGC ACA TCT TGG AAA GAA ATT CAT AGT GGG CTT AGT AAT GTT TTG AGA 1393
Arg Thr Ser Trp Lys Glu Ile His Ser Gly Leu Ser Asn Val Leu Arg
115 120 125

GCG GGG GTA ACG CCT ATT GTT GCT CAT ATT GAG CGC TAT GAT GCC CTC 1441
Ala Gly Val Thr Pro Ile Val Ala His Ile Glu Arg Tyr Asp Ala Leu
130 135 140

GAA GAA AAT GCT GAC CGT GTT CGA GAA ATC ATC AAT ATG GGC TGC TAT 1489
Glu Glu Asn Ala Asp Arg Val Arg Glu Ile Ile Asn Met Gly Cys Tyr
145 150 155

ACT CAA GTC AAT AGC TCA CAT GTC CTC AAA CCA AAG CTC TTT GGA GAT 1537
Thr Gln Val Asn Ser Ser His Val Leu Lys Pro Lys Leu Phe Gly Asp
160 165 170 175

AAA GAT AAA GTA AGA AAG AAA CGT GTT CGC TTT TTC TTG GAG AAA AAT 1585
Lys Asp Lys Val Arg Lys Lys Arg Val Arg Phe Phe Leu Glu Lys Asn
180 185 190

TTG GTT CAT ATG GTT GCT AGC GAC ATG CAT AAT CTT GGG CCG AGA CCA 1633
Leu Val His Met Val Ala Ser Asp Met His Asn Leu Gly Pro Arg Pro
195 200 205

CCA TTT ATG AAA GAT GCT TAT GAA ATT GTT AAA AAG AAC TAC GGC TCC 1681
Pro Phe Met Lys Asp Ala Tyr Glu Ile Val Lys Lys Asn Tyr Gly Ser
210 215 220

AAA CGT GCT AAG AAT CTT TTT ATT GAA AAT CCC AAA ACA TTA CTA GAA 1729
Lys Arg Ala Lys Asn Leu Phe Ile Glu Asn Pro Lys Thr Leu Leu Glu
225 230 235

AAT CAA TAT TTA TAGGAGATAT T ATG AAT CAA GAT AAC ACT AAA AGT GAT 1779
Asn Gln Tyr Leu Met Asn Gln Asp Asn Thr Lys Ser Asp
240 1 5

GAA ATC GAC GTA CTA GCA TTG CTA CAT AAA CTT TGG ACG AAG AAG CTT 1827
Glu Ile Asp Val Leu Ala Leu Leu His Lys Leu Trp Thr Lys Lys Leu
10 15 20 25

TTG ATT CTT TTC ACA GCT TTT TAT TTC GCT GTT TTC AGT TTC TTA GGT 1875
Leu Ile Leu Phe Thr Ala Phe Tyr Phe Ala Val Phe Ser Phe Leu Gly
30 35 40

ACT TAT TTC TTT ATC CAA CCA ACA TAT ACA TCA ACA ACG CGT ATC TAT 1923
Thr Tyr Phe Phe Ile Gln Pro Thr Tyr Thr Ser Thr Thr Arg Ile Tyr
45 50 55

GTT GTT AAT CAG GCA ACA GAT AAT AAG AAT CTT TCT GCT CAA GAT TTG 1971
Val Val Asn Gln Ala Thr Asp Asn Lys Asn Leu Ser Ala Gln Asp Leu
60 65 70

CAA GCT GGT ACC TAT TTG GCA AAT GAC TAT AAA GAG ATT ATT GCA TCA 2019
Gln Ala Gly Thr Tyr Leu Ala Asn Asp Tyr Lys Glu Ile Ile Ala Ser
75 80 85

AAT GAT GTA TTA TCA GAA GTT ATT AAA GAT GAA AAA TTG AAT TTG AGT 2067
Asn Asp Val Leu Ser Glu Val Ile Lys Asp Glu Lys Leu Asn Leu Ser
90 95 100 105

GAG GCA GAA CTG TCT AAA ATG GTT TCA GTT AAT ATT CCT ACT GAT ACT 2115
Glu Ala Glu Leu Ser Lys Met Val Ser Val Asn Ile Pro Thr Asp Thr
110 115 120

CGT CTT ATT TCA ATT TCT GTT AAT GCT AAA ACT GGT CAA GAT GCG CAA 2163
Arg Leu Ile Ser Ile Ser Val Asn Ala Lys Thr Gly Gln Asp Ala Gln
125 130 135

ACA CTT GCC AAT AAG GTT CGT GAA GTT GCT TCA AAA AAA ATC AAG AAG 2211
Thr Leu Ala Asn Lys Val Arg Glu Val Ala Ser Lys Lys Ile Lys Lys
140 145 150

GTG ACA AAA GTT GAA GAT GTC ACA ACG CTC GAA GAA GCT AAA TTG CCA 2259
Val Thr Lys Val Glu Asp Val Thr Thr Leu Glu Glu Ala Lys Leu Pro
155 160 165

GAG TCA CCA TCT TCA CCA AAT ATC AAA CTT AAT GTG CTT CTT GGG GCA 2307
Glu Ser Pro Ser Ser Pro Asn Ile Lys Leu Asn Val Leu Leu Gly Ala
170 175 180 185

GTG CTT GGA GGA TTC CTT GCA GTG GTT GGT GTA TTG GTA CGT GAA ATC 2355
Val Leu Gly Gly Phe Leu Ala Val Val Gly Val Leu Val Arg Glu Ile
190 195 200

CTA GAT GAT CGT GTT CGC CGT CCA GAA GAT GTG GAA GAT GCC CTT GGA 2403
Leu Asp Asp Arg Val Arg Arg Pro Glu Asp Val Glu Asp Ala Leu Gly
205 210 215

ATG ACA CTT CTT GGA ATT GTC CCT GAT ACA GAT AAA ATT TAAGGAGAAG 2452
Met Thr Leu Leu Gly Ile Val Pro Asp Thr Asp Lys Ile
220 225 230

AA ATG CCT TTA TTA AAG TTA GTT AAA TCA AAA GTA GAC TTT GCT AAA 2499
Met Pro Leu Leu Lys Leu Val Lys Ser Lys Val Asp Phe Ala Lys
1 5 10 15

AAG ACG GAA GAG TAT TAT AAC GCT ATT CGC ACA AAT ATT CAA TTT TCT     2547
Lys Thr Glu Glu Tyr Tyr Asn Ala Ile Arg Thr Asn Ile Gln Phe Ser
                20                  25                  30

GGT GCT CAG ATG AAA GTG ATT GCG ATT AGC TCT GTT GAA GCT GGT GAA     2595
Gly Ala Gln Met Lys Val Ile Ala Ile Ser Ser Val Glu Ala Gly Glu
            35                  40                  45

GGA AAA TCA ATG ATA TCT GTT AAC TTG GCG ATT TCA TTT GCT AGT GTT     2643
Gly Lys Ser Met Ile Ser Val Asn Leu Ala Ile Ser Phe Ala Ser Val
        50                  55                  60

GGG CTC CGA ACA CTT CTG ATT GAT GCG GAA ACG CGT AAT TCT GTT TTG     2691
Gly Leu Arg Thr Leu Leu Ile Asp Ala Glu Thr Arg Asn Ser Val Leu
    65                  70                  75

TCA GGT ACA TTT AAA TCA AAT GAG CCT TAT AAA GGT CTT TCA AAT TTC     2739
Ser Gly Thr Phe Lys Ser Asn Glu Pro Tyr Lys Gly Leu Ser Asn Phe
80                  85                  90                  95

CTT TCA GGA AAT GCC GAT CTA AAT GAA ACG ATT TGC CAA ACT GAT ATT     2787
Leu Ser Gly Asn Ala Asp Leu Asn Glu Thr Ile Cys Gln Thr Asp Ile
                100                 105                 110

TCT GGT TTA GAT GTT ATT GCA TCT GGT CCT GTT CCA CCT AAT CCA ACA     2835
Ser Gly Leu Asp Val Ile Ala Ser Gly Pro Val Pro Pro Asn Pro Thr
            115                 120                 125

AGT CTT TTG CAA AAT GAT AAT TTT AGA CAT TTG ATG GAA GTT GCT CGT     2883
Ser Leu Leu Gln Asn Asp Asn Phe Arg His Leu Met Glu Val Ala Arg
        130                 135                 140

AGT TGT TAT GAT TAT GTC ATC ATC GAT ACA CCA CCA GTT GGT CTG GTT     2931
Ser Cys Tyr Asp Tyr Val Ile Ile Asp Thr Pro Pro Val Gly Leu Val
    145                 150                 155

ATT GAT GCA GTT ATT ATT GCC CAT CAG GCT GAT GCC AGT CTT TTG GTT     2979
Ile Asp Ala Val Ile Ile Ala His Gln Ala Asp Ala Ser Leu Leu Val
160                 165                 170                 175

ACA GAA GCT GGG AAA ATT AAA CGT CGT TTC GTA ACT AAG GCC GTT GAA     3027
Thr Glu Ala Gly Lys Ile Lys Arg Arg Phe Val Thr Lys Ala Val Glu
                180                 185                 190

CAA TTG GTA GAA AGT GGT TCT CAG TTC TTA GGG GTC GTC CTT AAT AAA     3075
Gln Leu Val Glu Ser Gly Ser Gln Phe Leu Gly Val Val Leu Asn Lys
            195                 200                 205

GTT GAC ATG ACA GTT GAT AAA TAT GGA TTT TAT GGT TCT TAC GGA TCA     3123
Val Asp Met Thr Val Asp Lys Tyr Gly Phe Tyr Gly Ser Tyr Gly Ser
        210                 215                 220

TAT GGC GAG TAT GGA AAA AAA TCT GAC CAA AAA GAA GGT CAT TCA AGA     3171
Tyr Gly Glu Tyr Gly Lys Lys Ser Asp Gln Lys Glu Gly His Ser Arg
    225                 230                 235

GCA CAT CGT CGT AGA AAA GTC GGT TGG AAT TAACGCGTTA GTGTGTTTTA     3221
Ala His Arg Arg Arg Lys Val Gly Trp Asn
240                 245

AGATGTCGTT GGGAACGACA AGTGGAGGGA ATGAG ATG TCA CAA GCT AAA GAG     3274
                                       Met Ser Gln Ala Lys Glu
                                       1               5

GAA ATT TCA GAT GTT ATG ACT TAT TCA GAG CTA ACA AGT CAT AAG CCC     3322
Glu Ile Ser Asp Val Met Thr Tyr Ser Glu Leu Thr Ser His Lys Pro
            10                  15                  20

AAA ATT ATT TAT AGC TTG ATT AAG CGG ATT GGT GAT ATT TTG GTT AGT     3370
Lys Ile Ile Tyr Ser Leu Ile Lys Arg Ile Gly Asp Ile Leu Val Ser
        25                  30                  35

TCT ATT GGT TTA ATT ATT TTG ATA CCG CTA TTT TTG ATA GTT GCT TTG 3418
Ser Ile Gly Leu Ile Ile Leu Ile Pro Leu Phe Leu Ile Val Ala Leu
40 45 50

ATC ATG AAA TGC TCT GAA CCA ACA GCA CCT ATA TTT TTC TCA CAT ATT 3466
Ile Met Lys Cys Ser Glu Pro Thr Ala Pro Ile Phe Phe Ser His Ile
55 60 65 70

AGA AAT GGT AAA AAT GGC AAA AAG TTC AAA ATG TAT AAA TTT AGA ACC 3514
Arg Asn Gly Lys Asn Gly Lys Lys Phe Lys Met Tyr Lys Phe Arg Thr
75 80 85

ATG TGT CAG GAC GCA GAA TCG ATT TTG ATG AAA GAT ACG GAA CTT TTT 3562
Met Cys Gln Asp Ala Glu Ser Ile Leu Met Lys Asp Thr Glu Leu Phe
90 95 100

GCA AAA TTT AAG GCA AAT GGT TAT AAA CTT GAA ACG CAT GAA GAT CCT 3610
Ala Lys Phe Lys Ala Asn Gly Tyr Lys Leu Glu Thr His Glu Asp Pro
105 110 115

AGA ATT ACA AAA ATC GGT GGC ATA TTA AGG AAA ACA AGT ATT GAT GAA 3658
Arg Ile Thr Lys Ile Gly Gly Ile Leu Arg Lys Thr Ser Ile Asp Glu
120 125 130

TTG CCA CAA CTG ATT AAT GTT TTT TTA GGA CAA ATG TCA TTA GTG GGT 3706
Leu Pro Gln Leu Ile Asn Val Phe Leu Gly Gln Met Ser Leu Val Gly
135 140 145 150

CCA CGT CCA CTA CCA GAT AGA GAA ATC ATT GAA TAC GGT GAT AAC CAA 3754
Pro Arg Pro Leu Pro Asp Arg Glu Ile Ile Glu Tyr Gly Asp Asn Gln
155 160 165

GAA AAA TTT TTA AGC GTT AAA CCA GGC ATG ACA GGA TGG TGG CAA GTT 3802
Glu Lys Phe Leu Ser Val Lys Pro Gly Met Thr Gly Trp Trp Gln Val
170 175 180

TCA GGG AGA AGT ACT ATT GGG TAT CCT GAG CGG TGT CAT CTT GAG CTT 3850
Ser Gly Arg Ser Thr Ile Gly Tyr Pro Glu Arg Cys His Leu Glu Leu
185 190 195

TAT TAT GTA GAA AAG TGT TGT TTT ACT TTC GAT GTT CTT ATA TTA CTT 3898
Tyr Tyr Val Glu Lys Cys Cys Phe Thr Phe Asp Val Leu Ile Leu Leu
200 205 210

AAG ACA ATT GGG ATT GTT TTG AAG AGA GTT GGA GCG CGT TAGTACTGAT 3947
Lys Thr Ile Gly Ile Val Leu Lys Arg Val Gly Ala Arg
215 220 225

GAAACAAAAA TTATTATTGA TAATAGAAGC GATGAGTGGT GGAGCCGGTC GTCATGTACA 4007

GACTTGATTA GTCATCTACC TCAAGAAAAC TTTGATATTT ATGTGATTTA TTCAAATCAT 4067

AGAACAAATC CTGCGTTTTG GAAAAAATAG TAACG ATG AAT GAG CAA GTA ACT 4120
Met Asn Glu Gln Val Thr
1 5

TTT ATT TTA TGT GAT TTT GTC GTA AGA GAA ATT AAA CCG AAA TAT GAT 4168
Phe Ile Leu Cys Asp Phe Val Val Arg Glu Ile Lys Pro Lys Tyr Asp
10 15 20

TTG CTT GCT TAT CAA TTT ATT TCT AAA AAG ATT AAA GAA ATC AAA CCA 4216
Leu Leu Ala Tyr Gln Phe Ile Ser Lys Lys Ile Lys Glu Ile Lys Pro
25 30 35

GAT ATT GTA CAT TGT CAC AGT TCA AAA GCT GGT GTT ATT GGT CGT TTA 4264
Asp Ile Val His Cys His Ser Ser Lys Ala Gly Val Ile Gly Arg Leu
40 45 50

GCT GCC AAA AGA CGA GGT GTT AAA AAA ATA TTT TAT ACG CCA CAT GCT 4312
Ala Ala Lys Arg Arg Gly Val Lys Lys Ile Phe Tyr Thr Pro His Ala
55 60 65 70

TAT TCG TTT TTG GCA CCT GAA TTT AGT GGG AAG AAA AAG TTT CTA TTT 4360
Tyr Ser Phe Leu Ala Pro Glu Phe Ser Gly Lys Lys Lys Phe Leu Phe
75 80 85

GTT CAA ATT GAA AAG TTT TTA AGC CGA TTT GCG ACA ACT AAG ATA TTT 4408
Val Gln Ile Glu Lys Phe Leu Ser Arg Phe Ala Thr Thr Lys Ile Phe
90 95 100

TGT GTG TCA ATA GCG GAA ATG CAA GCT GCT CTT GAA GTA AAT CTA GAT 4456
Cys Val Ser Ile Ala Glu Met Gln Ala Ala Leu Glu Val Asn Leu Asp
105 110 115

AAA ACC GAT AAG TTT CAG GTA ATT TAT AAT GGT TTG CCA GAA ATT GAT 4504
Lys Thr Asp Lys Phe Gln Val Ile Tyr Asn Gly Leu Pro Glu Ile Asp
120 125 130

TTA CCA AGC AAA GAA ACG ATT CGG GCG CAA TTA GGA CTG GAA AAG GCA 4552
Leu Pro Ser Lys Glu Thr Ile Arg Ala Gln Leu Gly Leu Glu Lys Ala
135 140 145 150

GCA GTT GTT ATA GGC AAT AAT GCA AAA ATG TCG GAA CAG AAA AAT CCT 4600
Ala Val Val Ile Gly Asn Asn Ala Lys Met Ser Glu Gln Lys Asn Pro
155 160 165

ATG TTT TTT ATG GAA ATT GCC CGA AAA ATG ATT AGA CAA AAC GCA AAT 4648
Met Phe Phe Met Glu Ile Ala Arg Lys Met Ile Arg Gln Asn Ala Asn
170 175 180

TGG CAT TTT GTG TGG GTA GGT GAT GGT CAG CTG ATG CCA CTT TTT CAA 4696
Trp His Phe Val Trp Val Gly Asp Gly Gln Leu Met Pro Leu Phe Gln
185 190 195

TCA TTT ATT AAG CAA AAT GGA CTA GAG GGA AAT ATC CAT TTG CTT GGC 4744
Ser Phe Ile Lys Gln Asn Gly Leu Glu Gly Asn Ile His Leu Leu Gly
200 205 210

GAG CGT CCT GAT AGT GAA ATA GTT GTG ACA GCC TAT GAC ATC TTC TTG 4792
Glu Arg Pro Asp Ser Glu Ile Val Val Thr Ala Tyr Asp Ile Phe Leu
215 220 225 230

ACG ACT TCC CAA TAT GAA GGT TTA CCT TAT GCA CCA ATT GAA GCG ATG 4840
Thr Thr Ser Gln Tyr Glu Gly Leu Pro Tyr Ala Pro Ile Glu Ala Met
235 240 245

CGA GCT GGT GTC CCG ATT CTT GCG ACA AAA GTT GTT GGC AAT AGT GAG 4888
Arg Ala Gly Val Pro Ile Leu Ala Thr Lys Val Val Gly Asn Ser Glu
250 255 260

CTT GTG ATA GAG GGC AAA AAT GGT TAT TTG ATT GAC TTA GAG TGG TCA 4936
Leu Val Ile Glu Gly Lys Asn Gly Tyr Leu Ile Asp Leu Glu Trp Ser
265 270 275

AAA TCT GTC GAA GAA AAA TTA TAT AAG GCA GCG AAA ATA GAT GCA CAA 4984
Lys Ser Val Glu Glu Lys Leu Tyr Lys Ala Ala Lys Ile Asp Ala Gln
280 285 290

ATG ATT AAA GCA GAT TTT AGG CAA AGG TTT GCG ATT GAT CAG ATA TTA 5032
Met Ile Lys Ala Asp Phe Arg Gln Arg Phe Ala Ile Asp Gln Ile Leu
295 300 305 310

AAG CAA ATT GAA ACA ATT TAT TTA GCT TGAATGAAGA ATGAGGAGGC 5079
Lys Gln Ile Glu Thr Ile Tyr Leu Ala
315

ATAAATGCTG ATTTTGAAAT TAAAATTTCA TCTTAATTGG TACACAAACG AAAACCATTA 5139

TTACACGTGA GTATTCGAAG ACCTGGAAAC GAGGCGATGA GCCGTATTAT CCAGTGAACA 5199

ATGATCGTAA CAACAAACTC TATACTGCCT ATAAGCGTCT TGCCGAGCAA CAAGAGAATG 5259

TCATTTTCGG TGGACGTCTA GGTCACTACC GTTACTACGA TATGCACCAG GTAATTGGAG 5319

CTGCCTTGCA GTGTGTCAGA AATGAAGTGA AGTAAATCTT GATGAAGTTG AATAACTTTA 5379

AGTAATTTTA TACTTAATCC AATTGATGAA AATATTTTTG TATCGATTTA TCTTCTGTAA 5439

GAAGAGTCCT AATCGTTTAA AAAATGTACA ATTGAGTTTT TATATTTTTA AATAAAGTTA 5499

CTTTTAAGTC GTGTTATAGA ATATACATGA ATAGGTGTAT TAGAAAATTT ATTAATCTAA 5559

TCCTCGAAAA TAACTGACTG TAAGGAATCA AGTT GTG GAG TGT AAG TTG TCA 5611
Val Glu Cys Lys Leu Ser
1 5

AAT GGA GAG GAA AAT AAT ATG AAA AAA ATT TCA ATT TTA CAC TTT TCC 5659
Asn Gly Glu Glu Asn Asn Met Lys Lys Ile Ser Ile Leu His Phe Ser
10 15 20

CAA GTA TCA GGC GGG GGA GTT GAA AAG TAC ATA AAA TTA TTT TTA AAG 5707
Gln Val Ser Gly Gly Gly Val Glu Lys Tyr Ile Lys Leu Phe Leu Lys
25 30 35

TAT TCT GAT GTG ACA AAA TTT AAT AAT TAT TTA GTT GCA CCT AAT CTT 5755
Tyr Ser Asp Val Thr Lys Phe Asn Asn Tyr Leu Val Ala Pro Asn Leu
40 45 50

GAA AAT TAT GAC GAA TTT AAT GGA TAT TTA AAG ATG TCT GTC AAT TTT 5803
Glu Asn Tyr Asp Glu Phe Asn Gly Tyr Leu Lys Met Ser Val Asn Phe
55 60 65 70

AAT ATG GAA CAA ACT TTT TCT CCG CTA AAA ATA TTC AAA AAT GTC TTT 5851
Asn Met Glu Gln Thr Phe Ser Pro Leu Lys Ile Phe Lys Asn Val Phe
75 80 85

TTT ATT CGT AGT GTA CTC AAA AAA ATA AAC CCA GAT ATA GTA TAC CTA 5899
Phe Ile Arg Ser Val Leu Lys Lys Ile Asn Pro Asp Ile Val Tyr Leu
90 95 100

CAT AGT ACA TTT GCA GGT GTC GTA GGT CGT ATT GCT TCA ATA GGT TTG 5947
His Ser Thr Phe Ala Gly Val Val Gly Arg Ile Ala Ser Ile Gly Leu
105 110 115

CCA ACA AAA GTA GTA TAC AAT CCT CAC GGA TGG TCC TTC AAA ATG GAC 5995
Pro Thr Lys Val Val Tyr Asn Pro His Gly Trp Ser Phe Lys Met Asp
120 125 130

AAC AGC TAT TTG AAA AAG CTT ATT TTT AAA TTA ATC GAA TTT TCT TTA 6043
Asn Ser Tyr Leu Lys Lys Leu Ile Phe Lys Leu Ile Glu Phe Ser Leu
135 140 145 150

TCT TTT TTA ACT GAT AAG TTT ATT TTA ATT TCG GAA TCT GAG TAT ATT 6091
Ser Phe Leu Thr Asp Lys Phe Ile Leu Ile Ser Glu Ser Glu Tyr Ile
155 160 165

TTG GCT AAC CAT ATT TCA TTT AAT AAA AGC AAG TTT TCA CTA ATT AAT 6139
Leu Ala Asn His Ile Ser Phe Asn Lys Ser Lys Phe Ser Leu Ile Asn
170 175 180

AAT GGT GTT GAA GTG ATT ACA GGG GAT TCA AGA AAT GAG ATA GAA GAG 6187
Asn Gly Val Glu Val Ile Thr Gly Asp Ser Arg Asn Glu Ile Glu Glu
185 190 195

ATA TTT CCA AAT GAG GAT TTT ATA ATT GGC ATG GTT GGC AGA CTA AGC 6235
Ile Phe Pro Asn Glu Asp Phe Ile Ile Gly Met Val Gly Arg Leu Ser
200 205 210

CCA CCC AAA GAG TTT TTC TTT TTT ATT GAT TTT GCA AAA AAA ATA TTA 6283
Pro Pro Lys Glu Phe Phe Phe Phe Ile Asp Phe Ala Lys Lys Ile Leu
215 220 225 230

CAA ATT CGA AAC GAT ACC AAT TTT ATT ATC GTG GGT GAT GGA GAG TTA 6331
Gln Ile Arg Asn Asp Thr Asn Phe Ile Ile Val Gly Asp Gly Glu Leu
235 240 245

CGA AGT GAA ATA GAA AGA ATG ATA CTA GAT AAT GGG TTA GGA GAT AAA 6379
Arg Ser Glu Ile Glu Arg Met Ile Leu Asp Asn Gly Leu Gly Asp Lys
250 255 260

ATC TAT ATT ACT GGG TGG GTT GAT AAT CCG AGA AAC TAT ATA GAG AAG 6427
Ile Tyr Ile Thr Gly Trp Val Asp Asn Pro Arg Asn Tyr Ile Glu Lys
265 270 275

TTT GAT CAA GCT ATT CTG TTT TCT AGA TGG GAG GGT CTT AGC CTA ACG 6475
Phe Asp Gln Ala Ile Leu Phe Ser Arg Trp Glu Gly Leu Ser Leu Thr
280 285 290

ATT GCG GAA TAT ATG TCT CAG AAG AAA ACA ATT TTA GCA ACA AAT ATT 6523
Ile Ala Glu Tyr Met Ser Gln Lys Lys Thr Ile Leu Ala Thr Asn Ile
295 300 305 310

GGT GGC ATT AAT GAT TTA ATC ACT GAT GGT GAA ACA GGA ATG CTG ATT 6571
Gly Gly Ile Asn Asp Leu Ile Thr Asp Gly Glu Thr Gly Met Leu Ile
315 320 325

GAA GTT GGA GAC TTG AAT TCA GCA GTA TCT AAA TCT TTC GAG CTA AGA 6619
Glu Val Gly Asp Leu Asn Ser Ala Val Ser Lys Ser Phe Glu Leu Arg
330 335 340

AAT AAT AAA GAG GTT TCG AAT CAA TTA GCG AAT AAC GCT TAT AAT AAA 6667
Asn Asn Lys Glu Val Ser Asn Gln Leu Ala Asn Asn Ala Tyr Asn Lys
345 350 355

GTT GTT GAA CAG TTT TCG ATT GAA AAA CAG ATG GCT GAG ATA GAA AGT 6715
Val Val Glu Gln Phe Ser Ile Glu Lys Gln Met Ala Glu Ile Glu Ser
360 365 370

TTA TTT ATA GAG ATG TGT AAC AAT GAG AAA TAGAGACTTA AAGAAAATAC 6765
Leu Phe Ile Glu Met Cys Asn Asn Glu Lys
375 380

AGGTTATTTG ATTGACTTAG AGTGGTCAAA ATCTGTCGAA GAAAAATTAT ATAAGGCAGC 6825

GAAAATGGAT GCACAAATGA TTAAAGCAGA TTTTAGGCAA AGGTTTGCGA TTGATCAGAT 6885

GTTAAAGCAA ATTAAAACAA TTTATTTAGC TTGAATGAAG AAAGAGGAGG CATAA ATG 6943
Met
1

CTG ATT TTG AAA TTA AAA TTT CAT CTT AAA TCG TTA TTC CTT AAA TGG 6991
Leu Ile Leu Lys Leu Lys Phe His Leu Lys Ser Leu Phe Leu Lys Trp
5 10 15

ATT TAT CGA TTA CTT TAT CTA AAA AAG TTT CAG TTT GGT GCA CGC TTG 7039
Ile Tyr Arg Leu Leu Tyr Leu Lys Lys Phe Gln Phe Gly Ala Arg Leu
20 25 30

ACG TTT CGA GAT GGG TTT CAT TTG TTA ATT GAA AAA TCT GGG AAA GTT 7087
Thr Phe Arg Asp Gly Phe His Leu Leu Ile Glu Lys Ser Gly Lys Val
35 40 45

ATC ATC GGG AAT CAT GTT TTT TTT AAT AAC TTT TGT TCA ATT AAT GCC 7135
Ile Ile Gly Asn His Val Phe Phe Asn Asn Phe Cys Ser Ile Asn Ala
50 55 60 65

ATG TTA TCA GTA ACG ATT GGT GAT GAC TGT ATT TTT GGT GAA AAC GTT 7183
Met Leu Ser Val Thr Ile Gly Asp Asp Cys Ile Phe Gly Glu Asn Val
70 75 80

AAA ATT TAT GAT CAC AAT CAT TGT TAT CAA AAT AAA AGT CAA CCT ATT 7231
Lys Ile Tyr Asp His Asn His Cys Tyr Gln Asn Lys Ser Gln Pro Ile
85 90 95

TCA AAA CAA GGT TTT TCA ACT GCT GCT ATC CAG ATT GGT CGT AAC TGT 7279
Ser Lys Gln Gly Phe Ser Thr Ala Ala Ile Gln Ile Gly Arg Asn Cys
100 105 110

TGG ATA GGT AGT CAA GTG ACG ATT TTA AAA GGT GTA ACC ATA GGT GAT 7327
Trp Ile Gly Ser Gln Val Thr Ile Leu Lys Gly Val Thr Ile Gly Asp
115 120 125

AAT AGT ATC ATT GGT GCT GGT GTG GTA GTT TAT CAA GAT GTG CCA GAA 7375
Asn Ser Ile Ile Gly Ala Gly Val Val Val Tyr Gln Asp Val Pro Glu
130 135 140 145

AAT TCG ATT GTT TTA TCT AAT GGA GAA ATT AGA AAG CGT GGC 7417
Asn Ser Ile Val Leu Ser Asn Gly Glu Ile Arg Lys Arg Gly
150 155

TAATTAAA ATG TAT CTT AAA AGT CTA ATC TCT ATT GTT ATT CCA GTA TAT 7467
Met Tyr Leu Lys Ser Leu Ile Ser Ile Val Ile Pro Val Tyr
1 5 10

AAT GTA GAG AAA TAT TTA GAA AAA TGT TTG CAA TCT GTT CAA AAT CAG 7515
Asn Val Glu Lys Tyr Leu Glu Lys Cys Leu Gln Ser Val Gln Asn Gln
15 20 25 30

ACT TAC AAT AAT TTT GAA GTG ATT TTA GTG AAT GAT GGC TCA ACC GAT 7563
Thr Tyr Asn Asn Phe Glu Val Ile Leu Val Asn Asp Gly Ser Thr Asp
35 40 45

TCA TCA CTT TCA ATA TGC GAA AAA TTT GTT AAT CAG GAT AAA AGA TTT 7611
Ser Ser Leu Ser Ile Cys Glu Lys Phe Val Asn Gln Asp Lys Arg Phe
50 55 60

TCT GTT TTT TCT AAA GAA AAT GGT GGT ATG TCA TCT GCA CGA AAT TTT 7659
Ser Val Phe Ser Lys Glu Asn Gly Gly Met Ser Ser Ala Arg Asn Phe
65 70 75

GGA ATT AAA AAG GCT AAA GGA TCG TTT ATC ACA TTT GTA GAT AGT GAT 7707
Gly Ile Lys Lys Ala Lys Gly Ser Phe Ile Thr Phe Val Asp Ser Asp
80 85 90

GAC TAC ATA GTA AAA GAT TAT CTT TCT CAT TTG GTA GCT GGG ATA AAA 7755
Asp Tyr Ile Val Lys Asp Tyr Leu Ser His Leu Val Ala Gly Ile Lys
95 100 105 110

AGT GAG ACC TCT ATA GTT TGT TCA AAG TTT TTT CTT GTA GAT GAA AAA 7803
Ser Glu Thr Ser Ile Val Cys Ser Lys Phe Phe Leu Val Asp Glu Lys
115 120 125

GGA AGT TTA TTG ACT AAA AAA GAG GCA CCT AAA AAG AAA TCA GAA GTC 7851
Gly Ser Leu Leu Thr Lys Lys Glu Ala Pro Lys Lys Lys Ser Glu Val
130 135 140

GTT TCA ATT GAG GAA AGT ATT AAA ATT CTT CTG TTG CAA CAA AAT GGC 7899
Val Ser Ile Glu Glu Ser Ile Lys Ile Leu Leu Leu Gln Gln Asn Gly
145 150 155

TAT GAT CTC GCT GTC TGG GGA AAA TTA TAC CCC GTT TCT TTC TTT GAA 7947
Tyr Asp Leu Ala Val Trp Gly Lys Leu Tyr Pro Val Ser Phe Phe Glu
160 165 170

ACA ATT TCT TTC CCA GAA GGA AAA CTT TAC GAA GAT ATG GGA ACA ACT 7995
Thr Ile Ser Phe Pro Glu Gly Lys Leu Tyr Glu Asp Met Gly Thr Thr
175 180 185 190

TAC AAA TTA CTA AAA TTG GCA AGT GAA GTG GTC TTC TTG GAT GCG TAT 8043
Tyr Lys Leu Leu Lys Leu Ala Ser Glu Val Val Phe Leu Asp Ala Tyr
195 200 205

GAT TAT GCC TAC GTA CAG CGA CCT AAT AGT ATC ATG AAT AGT TCT TTT 8091
Asp Tyr Ala Tyr Val Gln Arg Pro Asn Ser Ile Met Asn Ser Ser Phe
210 215 220

AAT TTG AAA AAG TTG GAT ATA ATA GAA ATG GTT CAT GAA ATG GAA AAC 8139
Asn Leu Lys Lys Leu Asp Ile Ile Glu Met Val His Glu Met Glu Asn
225 230 235

GAT ATA TTA GCA CAG TTT CCA AAT TTA GCA TTA TAT GTT AAG AAT CGA     8187
Asp Ile Leu Ala Gln Phe Pro Asn Leu Ala Leu Tyr Val Lys Asn Arg
    240                 245                 250

GCA TTT GCC GCG GAA GTG AAA ATC TTT TTA GAG ATT CCA AAA GAA AAA     8235
Ala Phe Ala Ala Glu Val Lys Ile Phe Leu Glu Ile Pro Lys Glu Lys
255                 260                 265                 270

GAA TTT GAG CAA GCG CAA AAG CAA CTT TGG CAT GAT ATC AAA AAG AAT     8283
Glu Phe Glu Gln Ala Gln Lys Gln Leu Trp His Asp Ile Lys Lys Asn
                275                 280                 285

AGA AAA GCA CCA TTT ATG ACA AAA GGT GCT AGA TTG AAG AAT AGG CTC     8331
Arg Lys Ala Pro Phe Met Thr Lys Gly Ala Arg Leu Lys Asn Arg Leu
            290                 295                 300

GGA GCT AGT CTG TCG TTT TTA GGT AAA TCT TTA TTT TTG ACT ATT GGG     8379
Gly Ala Ser Leu Ser Phe Leu Gly Lys Ser Leu Phe Leu Thr Ile Gly
        305                 310                 315

AAG CAG TTA GTA GAT AGA TAATGATATT GAAAGCGATA CGATACAATC            8427
Lys Gln Leu Val Asp Arg
    320

GTAAACTTCT TTTGGTGTTG ACTAGGAGTT AGCTTGAAAT TTGAATATAA AGGAAGCAAC   8487

AC ATG GTA ATT TAT TTT TTA CTT TTC CCG ATG ATC GCA ATG ATT TAT      8534
   Met Val Ile Tyr Phe Leu Leu Phe Pro Met Ile Ala Met Ile Tyr
     1               5                  10                  15

TTA ATG ACA TTG CTC TTA CGG CAA AAA GCA CAA ATC CAA AAA ACG ATT     8582
Leu Met Thr Leu Leu Leu Arg Gln Lys Ala Gln Ile Gln Lys Thr Ile
                20                  25                  30

TTT TGT GTT CTT ACG TTT GGT ACA CTA GGC TTT ATT TCA GCA AGT CGT     8630
Phe Cys Val Leu Thr Phe Gly Thr Leu Gly Phe Ile Ser Ala Ser Arg
            35                  40                  45

GCA TCA AGT GTT GGG ACG GAC GTT ACT TTA TAC GAA AAT ATT TTT AAA     8678
Ala Ser Ser Val Gly Thr Asp Val Thr Leu Tyr Glu Asn Ile Phe Lys
        50                  55                  60

TCT ATA AAT TAC GGG ATA AGT GCT GAG AAT AAT TGG GGA TAC GTC ATC     8726
Ser Ile Asn Tyr Gly Ile Ser Ala Glu Asn Asn Trp Gly Tyr Val Ile
    65                  70                  75

TAT AAC AAG CTG ATT GGT AGT GTA TTT GGC TAT ACA GGA CAT GAA ATC     8774
Tyr Asn Lys Leu Ile Gly Ser Val Phe Gly Tyr Thr Gly His Glu Ile
80                  85                  90                  95

ACG GCC GCT AAT TCA GTT TTG ATT ACA ATA CTT ATT GGT ATT TTT ATT     8822
Thr Ala Ala Asn Ser Val Leu Ile Thr Ile Leu Ile Gly Ile Phe Ile
                100                 105                 110

TGG AAA GTA GCG GAA CAT TAT TTT GTT GCG ACG TTT TTA TAC ATT AGC     8870
Trp Lys Val Ala Glu His Tyr Phe Val Ala Thr Phe Leu Tyr Ile Ser
            115                 120                 125

TTG TTT TAT TAT GCT ACA AGT TTT AAT ATT TCA AGA CAA TTT ATT GCC     8918
Leu Phe Tyr Tyr Ala Thr Ser Phe Asn Ile Ser Arg Gln Phe Ile Ala
        130                 135                 140

ATG GGG CTT GTA TTG GTA GCA ATT TCT TTT GCT TTA GAT AAA AAG GTT     8966
Met Gly Leu Val Leu Val Ala Ile Ser Phe Ala Leu Asp Lys Lys Val
    145                 150                 155

ATG CCT TGG TTT ATC TTG ACA GTT TTG GCT ACC TTA TTT CAT GCG ACA     9014
Met Pro Trp Phe Ile Leu Thr Val Leu Ala Thr Leu Phe His Ala Thr
160                 165                 170                 175

GCA ATC GTT GCT TTT CCT GTC TAT TGG CTT ACA AAA GTA CAT TGG GAT 9062
Ala Ile Val Ala Phe Pro Val Tyr Trp Leu Thr Lys Val His Trp Asp
180 185 190

GTG AAA AAG ACA TTA AGT ATT TTT CCA ATC ACG ATT TTT GCA AGT TTT 9110
Val Lys Lys Thr Leu Ser Ile Phe Pro Ile Thr Ile Phe Ala Ser Phe
195 200 205

ATT TTT GAT GCT ATT TTA AAC ATT TTT GTA CGT TTT TTC CCA CAT TAT 9158
Ile Phe Asp Ala Ile Leu Asn Ile Phe Val Arg Phe Phe Pro His Tyr
210 215 220

GAG ATG TAT ATC ACT GGA ACA CAA TTT AAT ATT TCA GAT CAG GGG CAG 9206
Glu Met Tyr Ile Thr Gly Thr Gln Phe Asn Ile Ser Asp Gln Gly Gln
225 230 235

GGA CGT GTG GTT TTG GTC AAA ATA TTT ATC TTG CTC ATT TTG TTT ACT 9254
Gly Arg Val Val Leu Val Lys Ile Phe Ile Leu Leu Ile Leu Phe Thr
240 245 250 255

TTA TTC TTG TTT TAT AAA AAA AGC TAT GCT TTG ATT TCT GAA TGT CAT 9302
Leu Phe Leu Phe Tyr Lys Lys Ser Tyr Ala Leu Ile Ser Glu Cys His
260 265 270

CAA AGT TTG ATA GCT TTG ACA ACC GTT GGA TTA AGT ATC GGT ATT GTA 9350
Gln Ser Leu Ile Ala Leu Thr Thr Val Gly Leu Ser Ile Gly Ile Val
275 280 285

TTT TAT AAT AAT ATT TTA CTC AAT AGA ATA GAA ATG TTT TAT TCA ATT 9398
Phe Tyr Asn Asn Ile Leu Leu Asn Arg Ile Glu Met Phe Tyr Ser Ile
290 295 300

TTA AGC ATC GTA TTT ATT CCA ATT GCT ATA GAT TAC ATT AGT TTG AAA 9446
Leu Ser Ile Val Phe Ile Pro Ile Ala Ile Asp Tyr Ile Ser Leu Lys
305 310 315

TTT AAA CAA AAA GAT GCT GTG CGA CTA ATG CTG ACG ATA GGT ATT TTG 9494
Phe Lys Gln Lys Asp Ala Val Arg Leu Met Leu Thr Ile Gly Ile Leu
320 325 330 335

TTA ATT ACA CTT GTG CCT TAC TAT ATA CAG GTT AGC GGT AAT TAT TCA 9542
Leu Ile Thr Leu Val Pro Tyr Tyr Ile Gln Val Ser Gly Asn Tyr Ser
340 345 350

GGA ATA TTG CCT TAT GTT ATT CAA CAA TAAAAAATAA AGTTTAGAGA 9589
Gly Ile Leu Pro Tyr Val Ile Gln Gln
355 360

GGAAATA ATG GAG GAT AGA AAG AAA CAA GTA ATT TTG ATA CTA TCC CAC 9638
Met Glu Asp Arg Lys Lys Gln Val Ile Leu Ile Leu Ser His
1 5 10

AGA AAT ACT CTC GCT CTA AAA TCA ACA ATA GAG CTT TTG GAT TCT CAA 9686
Arg Asn Thr Leu Ala Leu Lys Ser Thr Ile Glu Leu Leu Asp Ser Gln
15 20 25 30

TAC TTT GAT TTC TTT CTT CAT ATA GAT AAA AAA AGT AGA ATT CAA GAT 9734
Tyr Phe Asp Phe Phe Leu His Ile Asp Lys Lys Ser Arg Ile Gln Asp
35 40 45

TTT TTT TAT TTA AAA AAA ATT ACA AAA TTC TCC ACT ATT CAT TTT TCA 9782
Phe Phe Tyr Leu Lys Lys Ile Thr Lys Phe Ser Thr Ile His Phe Ser
50 55 60

GAA AGA AAA AAT GTA CAT TGG GGA GGT TTT TCT ATG GTA GAA GCA ATG 9830
Glu Arg Lys Asn Val His Trp Gly Gly Phe Ser Met Val Glu Ala Met
65 70 75

TTT GCG CTA TTA GAA TGT GCA CGT GAT ACA GGA GAA TAT TCT TAT TTT 9878
Phe Ala Leu Leu Glu Cys Ala Arg Asp Thr Gly Glu Tyr Ser Tyr Phe
80 85 90

CAT TTT TTA TCT GGA GAT GAT ATG CCA ATC AAA GAT AAT GAA ATA GTA 9926
His Phe Leu Ser Gly Asp Asp Met Pro Ile Lys Asp Asn Glu Ile Val
95 100 105 110

TTT AAT TTT TTT GAA AAT AGT TAT CCT AAA AAT TTT ATT GAT ATT CTA 9974
Phe Asn Phe Phe Glu Asn Ser Tyr Pro Lys Asn Phe Ile Asp Ile Leu
115 120 125

GAT TTT GAA AAT GTC AAT AAA AAT TCA TAT TTC TAC GAA CCC CCT GAG 10022
Asp Phe Glu Asn Val Asn Lys Asn Ser Tyr Phe Tyr Glu Pro Pro Glu
130 135 140

ATG ATA GAG GAG AGA GTG AAG TAC TAC TAT CCT CAT ATG GAT ATT CTA 10070
Met Ile Glu Glu Arg Val Lys Tyr Tyr Tyr Pro His Met Asp Ile Leu
145 150 155

AAC AGA AAA GGA ACA AAT TTC ATA GGG AAA AAA CTA ATT TAT CTA CAA 10118
Asn Arg Lys Gly Thr Asn Phe Ile Gly Lys Lys Leu Ile Tyr Leu Gln
160 165 170

AAA TTG TTG AAA GTT AAT CGC TTG AAA AAT AGA GAG ATA GAA ATT TTC 10166
Lys Leu Leu Lys Val Asn Arg Leu Lys Asn Arg Glu Ile Glu Ile Phe
175 180 185 190

AAG GGT CAT CAA TGG TGT AGT TTG ACA AAT CAA TTT GTA GAT ATT TTA 10214
Lys Gly His Gln Trp Cys Ser Leu Thr Asn Gln Phe Val Asp Ile Leu
195 200 205

TTG GAT AAA GAG GAA AGA AGA GTA GGT AAG TCT TAT TTT TCA TCT AGT 10262
Leu Asp Lys Glu Glu Arg Arg Val Gly Lys Ser Tyr Phe Ser Ser Ser
210 215 220

TTA ATA CCA GAT GAA TGT TAT TTT CAA ACG TTT GCT ATG ATA AAA AAA 10310
Leu Ile Pro Asp Glu Cys Tyr Phe Gln Thr Phe Ala Met Ile Lys Lys
225 230 235

GTT GAA ATT TAT CAA CAG AAA AAT ATG TCA GCA CGC TTA ATT GAT TGG 10358
Val Glu Ile Tyr Gln Gln Lys Asn Met Ser Ala Arg Leu Ile Asp Trp
240 245 250

ACA AGA GGG AAA CCA TAT ATT TGG CGA CAG GAT GAT TTT TTT GAA ATT 10406
Thr Arg Gly Lys Pro Tyr Ile Trp Arg Gln Asp Asp Phe Phe Glu Ile
255 260 265 270

ATG AAT GAT AAA GAT TCA ATG TTT TCT AGG AAG TTT GAT GAA AAT GTA 10454
Met Asn Asp Lys Asp Ser Met Phe Ser Arg Lys Phe Asp Glu Asn Val
275 280 285

GAT CGT AAA ATA ATT GAA GAA ATT TAT ATA AAA ATA AGA GGA AGA AGT 10502
Asp Arg Lys Ile Ile Glu Glu Ile Tyr Ile Lys Ile Arg Gly Arg Ser
290 295 300

ACT GAT GAA GCA AAT AAA ATC AAA GAT AAG AGA TTT ACA AAA 10544
Thr Asp Glu Ala Asn Lys Ile Lys Asp Lys Arg Phe Thr Lys
305 310 315

TAATTTTACC TATGTTTTTG GAAAGAAAAC TTTTCTTGGA AGGGGAGAAG CGATTATCAT 10604

AGATGAACCT GAGCATGGAA ATTTGGGAGA TCAAGCAATT GCTTTTGCAG AAAATCAATT 10664

TTTAGTAAAT CATGTATCAG TACGAGATGT AGAACATCTT ATAGAAAGCA AAACTATTTC 10724

AGAAATAAAA TCTATAAAAA AAAATATTGG AAAAAAAGAA TTAGTTTTTT TTCATGGGGG 10784

AGGAAATTTC GGGACACTTT ATCTAAAGTA TGAGCGCATT AGAAGATTGG CAGTATCAAA 10844

GCTTCCCTTT AATAAAATGA TTCTATTTCC TCAGTCAATT TCATTTGAAG ATAGTAGGTT 10904

TGGTCAGAAG CAGCTGAATA AAAGTAAAAA AATATACAGT CAAAATACAA ATTTTATTTT 10964

GACTGCAAGA GAACCAAAAT CTTATGGTTT AATGAAGAAA TGTTTTCCAT ATAACAAAGT 11024

AATCTTGACA CCGGATATCG TGCTCTCATT TAAATTTGAA GTCACCATTT CTGATACGCA 11084

TATTGGGAAA GAAAAGGATA GTGTTATAAC TTATGAAAAT CGTCAACACT ATCTTGAGAT 11144

AAAGTGGGAT GAAATTGCGC AGCATGAGGT CGCCTTAACT GATAGATTAC ATGGTATGAT 11204

TTTTTCATAT ATCACAGGCA CACCATGTGT TGTTTTGGCT AATAATAATC ATAAAATTGA 11264

AGGAACATAC AAACATTGGT TGAATGAAGT CAACTATATT CGTTTTATTG AAAATCCGAC 11324

TGTTGAAAAT ATTTTAGATG CAATCAATGA CTTAAAGCAA ATCGAACCTC ACTATATTGA 11384

TTTATCTGAT AAATTTCAAC CACTAATTGA TGCGATAAAA GGGTAAAGGT TTA ATG 11440
Met
1

AAT AAA TAT AAA AAA CTA CTA TCC AAC TCT CTT GTT TTC ACG ATA GGA 11488
Asn Lys Tyr Lys Lys Leu Leu Ser Asn Ser Leu Val Phe Thr Ile Gly
5 10 15

AAC TTA GGC AGC AAA CTG TTA GTC TTT TTA CTC GTA CCG CTC TAC ACC 11536
Asn Leu Gly Ser Lys Leu Leu Val Phe Leu Leu Val Pro Leu Tyr Thr
20 25 30

TAT GCG ATG ACA CCG CAA GAG TAT GGT ATG GCA GAC TTA TAT CAA ACA 11584
Tyr Ala Met Thr Pro Gln Glu Tyr Gly Met Ala Asp Leu Tyr Gln Thr
35 40 45

ACA GCA AAT CTA CTT TTG CCA TTA ATT ACA ATG AAT GTA TTT GAT GCA 11632
Thr Ala Asn Leu Leu Leu Pro Leu Ile Thr Met Asn Val Phe Asp Ala
50 55 60 65

ACT TTA CGT TTT GCT ATG GAA AAG TCA ATG ACA AAA GAG AGT GTG TTA 11680
Thr Leu Arg Phe Ala Met Glu Lys Ser Met Thr Lys Glu Ser Val Leu
70 75 80

ACA AAT TCT CTT GTG GTT TGG TGT TTT AGC GCG GTG TTC ACT TGT TTG 11728
Thr Asn Ser Leu Val Val Trp Cys Phe Ser Ala Val Phe Thr Cys Leu
85 90 95

GGC GCT TGT ATT ATC TAT GCG TTG AAC TTG AGT AAT AAA TGG TAT TTA 11776
Gly Ala Cys Ile Ile Tyr Ala Leu Asn Leu Ser Asn Lys Trp Tyr Leu
100 105 110

GCT TTA CTT TTA ACC TTC AAC TTA TTT CAA GGT GGA CAA AGT ATA TTA 11824
Ala Leu Leu Leu Thr Phe Asn Leu Phe Gln Gly Gly Gln Ser Ile Leu
115 120 125

AGC CAG TAT GCT AGA GGT ATA GGA AAG TCG AAA ATA TTT GCA GCT GGC 11872
Ser Gln Tyr Ala Arg Gly Ile Gly Lys Ser Lys Ile Phe Ala Ala Gly
130 135 140 145

GGA GTT ATT TTA ACC TTT TTG ACA GGC GCT TTA AAT ATT CTT TTT TTG 11920
Gly Val Ile Leu Thr Phe Leu Thr Gly Ala Leu Asn Ile Leu Phe Leu
150 155 160

GTA TAT TTA CCG CTT GGG ATT ACG GGC TAT TTA ATG TCC CTG GTT TTA 11968
Val Tyr Leu Pro Leu Gly Ile Thr Gly Tyr Leu Met Ser Leu Val Leu
165 170 175

GCG AAT GTA GGT ACG ATT CTA TTT TTT GCT GGC ACA CTT TCC ATT TGG 12016
Ala Asn Val Gly Thr Ile Leu Phe Phe Ala Gly Thr Leu Ser Ile Trp
180 185 190

AAG GAA ATT AGT TTT AAA ATA ATT GAT AAA AAA CTG ATT TGG CAA ATG 12064
Lys Glu Ile Ser Phe Lys Ile Ile Asp Lys Lys Leu Ile Trp Gln Met
195 200 205

CTC TAT TAT GCC TTA CCT TTG ATT CCT AGT TCC ATC CTG TGG TGG TTA 12112
Leu Tyr Tyr Ala Leu Pro Leu Ile Pro Ser Ser Ile Leu Trp Trp Leu
210 215 220 225

CTG AAT GCT TCT AGT CGC TAT TTC GTT TTA TTC TTT TTA GGA GCA GGT 12160
Leu Asn Ala Ser Ser Arg Tyr Phe Val Leu Phe Phe Leu Gly Ala Gly
230 235 240

GCT AAT GGT CTT TTG GCG GTC GCT ACC AAA ATT CCA AGT ATT ATT TCC 12208
Ala Asn Gly Leu Leu Ala Val Ala Thr Lys Ile Pro Ser Ile Ile Ser
245 250 255

ATT TTT AAT ACG ATT TTT ACA CAG GCG TGG CAA ATT TCA GCC ATA GAA 12256
Ile Phe Asn Thr Ile Phe Thr Gln Ala Trp Gln Ile Ser Ala Ile Glu
260 265 270

GAA TAT GAT TCT CAT CAA AAA TCA AAA TAT TAT TCG GAT GTT TTT CAC 12304
Glu Tyr Asp Ser His Gln Lys Ser Lys Tyr Tyr Ser Asp Val Phe His
275 280 285

TAC TTA GCA ACT TTT CTA TTG TTA GGG ACA TCA GCT TTT ATG ATT GTG 12352
Tyr Leu Ala Thr Phe Leu Leu Leu Gly Thr Ser Ala Phe Met Ile Val
290 295 300 305

CTT AAA CCA ATT GTC GAA AAA GTC GTT TCA AGT GAC TAT GCA AGT TCA 12400
Leu Lys Pro Ile Val Glu Lys Val Val Ser Ser Asp Tyr Ala Ser Ser
310 315 320

TGG CAA TAT GTT CCT TTC TTT ATG TTG TCG ATG CTA TTT TCC TCG TTT 12448
Trp Gln Tyr Val Pro Phe Phe Met Leu Ser Met Leu Phe Ser Ser Phe
325 330 335

TCT GAT TTT TTT GGG ACT AAT TAT ATT GCG GCT AAA CAA ACA AAA GGC 12496
Ser Asp Phe Phe Gly Thr Asn Tyr Ile Ala Ala Lys Gln Thr Lys Gly
340 345 350

GTA TTT ATG ACA TCT ATC TAT GGT ACC ATT GTT TGT GTC TTA CTC CAA 12544
Val Phe Met Thr Ser Ile Tyr Gly Thr Ile Val Cys Val Leu Leu Gln
355 360 365

GTG GTG CTG CTA CCC ATC ATC GGC TTG GAT GGC GCA GGT TTA TCA GCC 12592
Val Val Leu Leu Pro Ile Ile Gly Leu Asp Gly Ala Gly Leu Ser Ala
370 375 380 385

ATG CTT GGA TTT TTA ACA ACG TTT TTA TTG CGT GTC AAA GAT ACG CAA 12640
Met Leu Gly Phe Leu Thr Thr Phe Leu Leu Arg Val Lys Asp Thr Gln
390 395 400

AAA TTT GTG GTG ATT CAG ATT AAG TGG CGG ATT TTT ATC AGT AAT TTA 12688
Lys Phe Val Val Ile Gln Ile Lys Trp Arg Ile Phe Ile Ser Asn Leu
405 410 415

TTG ATC GTT TTT GGC ACA AAT TTT ATG TTT GTT TTA TCT ACC GAG 12733
Leu Ile Val Phe Gly Thr Asn Phe Met Phe Val Leu Ser Thr Glu
420 425 430

TGAATTTTTG TATTTTGGTC TTGCCCTATT ATTTTGTGGC ATGTTAGTGG TTAATAGCGT 12793

ACAATTTTAT ACATTATCAT GGCGCTAAAA ATAAAAAATA AGACATTTGG AATGAAATCC 12853

CCATAAAAAT AGACAGAAGG TGTATCTCGA ATGGTATCGA GATATATCTC CTGTCTATTT 12913

TTATGATACT TTTGTGTTAG CTCAACTCAA CCGCCTTTTA ATCTCCCAAC AACAATAATA 12973

CCCAATCAAA CAACCCAAAA AATTCAAGAT AATATCACTA ATGGCAAATG TGCCCAAATA 13033

AAAGATAAAT TGAATGGTTT CAATTACTAA AAGAGTGACC AAACTGACAA TGACAAACTG 13093

TTTGAAATCA GTATTGATAC AGTAAAGGCC ACCTAAAGGA ATGAAGTAGA TAATATTTAG 13153

CACAGCCTCT TGAATCGTTC TGGGATCCGC TTTTATAAAG TCAAAAGGAT TCAGTGACAT 13213

CGCCTGAAAA TCCGTTATTT TAGTAAAAAG TACCATGAAT AACAGTAATA AATACACACT 13273

GAAAGCAAGA TAGAGATAAA TAACTGAAAA ATATTTGAGG TGATACTGGA TACCAAACAA 13333

CCAGATAATC AGCGTTAATA AGAGTATTAA AGTCAATGTG GTATAGTCAA AGTGGTTAAT 13393

CAACTTAGCC AGGCTTTGAT AGCGAGTGAG AACGGGCATA ATCAGCCAAG TAATCGTCGC 13453

ATAACTCAGG ATAAATGTGA TCAATAAACT GCTGAGGTAG ATCATATATT TTCGCAACTG 13513

TTTCTAACTC CTTTTCTTGA TGAGATTAAC CCTATTTTAA CATATTTTAA AACTGTCATG 13573

TTTTTATGAA TTTAAAATAA ATGTTAAAGA AAATAAAAAT TCACCAGTTG GTTCTGTTGC 13633

AAAGTTTTCC AAAAAATCTA TTTTAGTGTA AAATTGAGAA AAAAGACAGA GAGGACAGAG 13693

TAATGAATTA TTTTAAAGGC AAACAATTCA AAAAAGACGT CATTATTGTC TCTGTTGGTT 13753

ACTACCTGCG TTACAATCTA AGCTATCGTT AAGTTCAGGA ATTGTTATAT GATC 13807

(2) INFORMATIONS POUR LA SEQ ID NO: 2:
(i) CARACTERISTIQUES DE LA SEQUENCE:
(A) LONGUEUR: 334 acides aminés
(B) TYPE: acide aminé
(D) CONFIGURATION: linéaire
(ii) TYPE DE MOLECULE: prot,ine
(xi) DESCRIPTION DE LA SEQUENCE: SEQ ID NO: 2:

Met Ser Ala Leu Lys Lys Asp Lys Lys Val Asp Val Lys Val Asp Asp
1               5                   10                  15

Val Ala Ser Tyr Gln Glu Ala Tyr Asp Asn Leu Lys Ser Gly Lys Ser
            20                  25                  30

Lys Ala Met Val Leu Ser Gly Ser Tyr Ala Ser Leu Leu Glu Ser Val
        35                  40                  45

Asp Ser Asn Tyr Ala Ser Asn Leu Lys Thr Ile Tyr Thr Tyr Lys Ile
    50                  55                  60

Lys Lys Lys Asn Ser Asn Ser Ala Asn Gln Val Asp Ser Arg Val Phe
65                  70                  75                  80

Asn Ile Tyr Ile Ser Gly Ile Asp Thr Tyr Gly Pro Ile Ser Thr Val
                85                  90                  95

Ser Arg Ser Asp Val Asn Ile Ile Met Thr Val Asn Met Asn Thr His
            100                 105                 110

Lys Ile Leu Leu Thr Thr Thr Pro Arg Asp Ala Tyr Val Lys Ile Pro
        115                 120                 125

Gly Gly Gly Ala Asp Gln Tyr Asp Lys Leu Thr His Ala Gly Ile Tyr
    130                 135                 140

Gly Val Glu Thr Ser Glu Gln Thr Leu Glu Asp Leu Tyr Gly Ile Lys
145                 150                 155                 160

Leu Asp Tyr Tyr Ala Arg Ile Asn Phe Thr Ser Phe Leu Lys Leu Ile
                165                 170                 175

Asp Gln Leu Gly Gly Val Thr Val His Asn Asp Gln Ala Phe Thr Gln
            180                 185                 190

Glu Lys Phe Asp Phe Pro Val Gly Asp Ile Gln Met Asn Ser Glu Gln
        195                 200                 205

Ala Leu Gly Phe Val Arg Glu Arg Tyr Asn Leu Asp Gly Gly Asp Asn
    210                 215                 220

Asp Arg Gly Lys Asn Gln Glu Lys Val Ile Ser Ala Ile Leu Asn Lys
225                 230                 235                 240

Leu Ala Ser Leu Lys Ser Val Ser Asn Phe Thr Ser Ile Val Asn Asn
                245                 250                 255

Leu Gln Asp Ser Val Gln Thr Asn Met Ser Leu Asn Thr Ile Asn Ala
            260                 265                 270

Leu Ala Asn Thr Gln Leu Glu Ser Gly Ser Lys Phe Thr Val Thr Ser
        275                 280                 285

Gln Ala Val Thr Gly Thr Gly Ser Thr Gly Gln Leu Ile Ser Tyr Ala
    290                 295                 300

Met Pro Asn Ser Ser Leu Tyr Met Met Lys Leu Asp Asn Ser Ser Val
305                 310                 315                 320

Glu Ser Ala Ser Gln Ala Ile Lys Lys Leu Met Glu Glu Lys
                325                 330

(2) INFORMATIONS POUR LA SEQ ID NO: 3:
(i) CARACTERISTIQUES DE LA SEQUENCE:
(A) LONGUEUR: 243 acides aminés
(B) TYPE: acide aminé
(D) CONFIGURATION: linéaire
(ii) TYPE DE MOLECULE: protéine
(xi) DESCRIPTION DE LA SEQUENCE: SEQ ID NO: 3:

Val Ile Asp Val His Ser His Ile Val Phe Asp Val Asp Asp Gly Pro
1               5                   10                  15

Glu Thr Leu Glu Glu Ser Leu Asp Leu Ile Gly Glu Ser Tyr Ala Gln
            20                  25                  30

Gly Val Arg Lys Ile Val Ser Thr Ser His Arg Arg Lys Gly Met Phe
        35                  40                  45

Glu Thr Pro Glu Asp Lys Ile Phe Ala Asn Phe Lys Lys Val Lys Ala
    50                  55                  60

Glu Ala Glu Ala Leu Tyr Pro Asp Leu Thr Ile Tyr Tyr Gly Gly Glu
65                  70                  75                  80

Leu Tyr Tyr Thr Ser Asp Ile Val Glu Lys Leu Glu Lys Asn Leu Ile
                85                  90                  95

Pro Arg Met His Asn Thr Gln Phe Ala Leu Ile Glu Phe Ser Ala Arg
            100                 105                 110

Thr Ser Trp Lys Glu Ile His Ser Gly Leu Ser Asn Val Leu Arg Ala
        115                 120                 125

Gly Val Thr Pro Ile Val Ala His Ile Glu Arg Tyr Asp Ala Leu Glu
    130                 135                 140

Glu Asn Ala Asp Arg Val Arg Glu Ile Ile Asn Met Gly Cys Tyr Thr
145                 150                 155                 160

Gln Val Asn Ser Ser His Val Leu Lys Pro Lys Leu Phe Gly Asp Lys
                165                 170                 175

Asp Lys Val Arg Lys Lys Arg Val Arg Phe Phe Leu Glu Lys Asn Leu
            180                 185                 190

Val His Met Val Ala Ser Asp Met His Asn Leu Gly Pro Arg Pro Pro
        195                 200                 205

Phe Met Lys Asp Ala Tyr Glu Ile Val Lys Lys Asn Tyr Gly Ser Lys
    210                 215                 220

Arg Ala Lys Asn Leu Phe Ile Glu Asn Pro Lys Thr Leu Leu Glu Asn
225                 230                 235                 240

Gln Tyr Leu

(2) INFORMATIONS POUR LA SEQ ID NO: 4:
    (i) CARACTERISTIQUES DE LA SEQUENCE:
        (A) LONGUEUR: 230 acides aminés
        (B) TYPE: acide aminé
        (D) CONFIGURATION: linéaire
    (ii) TYPE DE MOLECULE: protéine
    (xi) DESCRIPTION DE LA SEQUENCE: SEQ ID NO: 4:

Met Asn Gln Asp Asn Thr Lys Ser Asp Glu Ile Asp Val Leu Ala Leu
  1               5                  10                  15

Leu His Lys Leu Trp Thr Lys Lys Leu Leu Ile Leu Phe Thr Ala Phe
             20                  25                  30

Tyr Phe Ala Val Phe Ser Phe Leu Gly Thr Tyr Phe Phe Ile Gln Pro
         35                  40                  45

Thr Tyr Thr Ser Thr Thr Arg Ile Tyr Val Val Asn Gln Ala Thr Asp
     50                  55                  60

Asn Lys Asn Leu Ser Ala Gln Asp Leu Gln Ala Gly Thr Tyr Leu Ala
 65                  70                  75                  80

Asn Asp Tyr Lys Glu Ile Ile Ala Ser Asn Asp Val Leu Ser Glu Val
                 85                  90                  95

Ile Lys Asp Glu Lys Leu Asn Leu Ser Glu Ala Glu Leu Ser Lys Met
            100                 105                 110

Val Ser Val Asn Ile Pro Thr Asp Thr Arg Leu Ile Ser Ile Ser Val
        115                 120                 125

Asn Ala Lys Thr Gly Gln Asp Ala Gln Thr Leu Ala Asn Lys Val Arg
    130                 135                 140

Glu Val Ala Ser Lys Lys Ile Lys Lys Val Thr Lys Val Glu Asp Val
145                 150                 155                 160

Thr Thr Leu Glu Glu Ala Lys Leu Pro Glu Ser Pro Ser Ser Pro Asn
                165                 170                 175

Ile Lys Leu Asn Val Leu Leu Gly Ala Val Leu Gly Gly Phe Leu Ala
            180                 185                 190

Val Val Gly Val Leu Val Arg Glu Ile Leu Asp Asp Arg Val Arg Arg
        195                 200                 205

Pro Glu Asp Val Glu Asp Ala Leu Gly Met Thr Leu Leu Gly Ile Val
    210                 215                 220

Pro Asp Thr Asp Lys Ile
225                 230

(2) INFORMATIONS POUR LA SEQ ID NO: 5:
    (i) CARACTERISTIQUES DE LA SEQUENCE:
        (A) LONGUEUR: 249 acides aminés
        (B) TYPE: acide aminé
        (D) CONFIGURATION: linéaire
    (ii) TYPE DE MOLECULE: protéine
    (xi) DESCRIPTION DE LA SEQUENCE: SEQ ID NO: 5:

Met Pro Leu Leu Lys Leu Val Lys Ser Lys Val Asp Phe Ala Lys Lys
1               5                   10                  15

Thr Glu Glu Tyr Tyr Asn Ala Ile Arg Thr Asn Ile Gln Phe Ser Gly
            20                  25                  30

Ala Gln Met Lys Val Ile Ala Ile Ser Ser Val Glu Ala Gly Glu Gly
        35                  40                  45

Lys Ser Met Ile Ser Val Asn Leu Ala Ile Ser Phe Ala Ser Val Gly
    50                  55                  60

Leu Arg Thr Leu Leu Ile Asp Ala Glu Thr Arg Asn Ser Val Leu Ser
65                  70                  75                  80

Gly Thr Phe Lys Ser Asn Glu Pro Tyr Lys Gly Leu Ser Asn Phe Leu
                85                  90                  95

Ser Gly Asn Ala Asp Leu Asn Glu Thr Ile Cys Gln Thr Asp Ile Ser
            100                 105                 110

Gly Leu Asp Val Ile Ala Ser Gly Pro Val Pro Pro Asn Pro Thr Ser
        115                 120                 125

Leu Leu Gln Asn Asp Asn Phe Arg His Leu Met Glu Val Ala Arg Ser
    130                 135                 140

Cys Tyr Asp Tyr Val Ile Ile Asp Thr Pro Pro Val Gly Leu Val Ile
145                 150                 155                 160

Asp Ala Val Ile Ile Ala His Gln Ala Asp Ala Ser Leu Leu Val Thr
                165                 170                 175

Glu Ala Gly Lys Ile Lys Arg Arg Phe Val Thr Lys Ala Val Glu Gln
            180                 185                 190

Leu Val Glu Ser Gly Ser Gln Phe Leu Gly Val Val Leu Asn Lys Val
        195                 200                 205

Asp Met Thr Val Asp Lys Tyr Gly Phe Tyr Gly Ser Tyr Gly Ser Tyr
    210                 215                 220

Gly Glu Tyr Gly Lys Lys Ser Asp Gln Lys Glu Gly His Ser Arg Ala
225                 230                 235                 240

His Arg Arg Arg Lys Val Gly Trp Asn
                245

(2) INFORMATIONS POUR LA SEQ ID NO: 6:
    (i) CARACTERISTIQUES DE LA SEQUENCE:
          (A) LONGUEUR: 227 acides aminés
          (B) TYPE: acide aminé
          (D) CONFIGURATION: linéaire
    (ii) TYPE DE MOLECULE: protéine
    (xi) DESCRIPTION DE LA SEQUENCE: SEQ ID NO: 6:

Met Ser Gln Ala Lys Glu Glu Ile Ser Asp Val Met Thr Tyr Ser Glu
1               5                   10                  15

Leu Thr Ser His Lys Pro Lys Ile Ile Tyr Ser Leu Ile Lys Arg Ile
            20                  25                  30

Gly Asp Ile Leu Val Ser Ser Ile Gly Leu Ile Ile Leu Ile Pro Leu
        35                  40                  45

Phe Leu Ile Val Ala Leu Ile Met Lys Cys Ser Glu Pro Thr Ala Pro
    50                  55                  60

Ile Phe Phe Ser His Ile Arg Asn Gly Lys Asn Gly Lys Lys Phe Lys
65                  70                  75                  80

Met Tyr Lys Phe Arg Thr Met Cys Gln Asp Ala Glu Ser Ile Leu Met
                85                  90                  95

Lys Asp Thr Glu Leu Phe Ala Lys Phe Lys Ala Asn Gly Tyr Lys Leu
            100                 105                 110

Glu Thr His Glu Asp Pro Arg Ile Thr Lys Ile Gly Gly Ile Leu Arg
        115                 120                 125

Lys Thr Ser Ile Asp Glu Leu Pro Gln Leu Ile Asn Val Phe Leu Gly
    130                 135                 140

Gln Met Ser Leu Val Gly Pro Arg Pro Leu Pro Asp Arg Glu Ile Ile
145                 150                 155                 160

Glu Tyr Gly Asp Asn Gln Glu Lys Phe Leu Ser Val Lys Pro Gly Met
                165                 170                 175

Thr Gly Trp Trp Gln Val Ser Gly Arg Ser Thr Ile Gly Tyr Pro Glu
            180                 185                 190

Arg Cys His Leu Glu Leu Tyr Tyr Val Glu Lys Cys Cys Phe Thr Phe
        195                 200                 205

Asp Val Leu Ile Leu Leu Lys Thr Ile Gly Ile Val Leu Lys Arg Val
    210                 215                 220

Gly Ala Arg
225

(2) INFORMATIONS POUR LA SEQ ID NO: 7:
    (i) CARACTERISTIQUES DE LA SEQUENCE:
        (A) LONGUEUR: 319 acides aminés
        (B) TYPE: acide aminé
        (D) CONFIGURATION: linéaire
    (ii) TYPE DE MOLECULE: protéine
    (xi) DESCRIPTION DE LA SEQUENCE: SEQ ID NO: 7:

Met Asn Glu Gln Val Thr Phe Ile Leu Cys Asp Phe Val Val Arg Glu
1               5                   10                  15

Ile Lys Pro Lys Tyr Asp Leu Leu Ala Tyr Gln Phe Ile Ser Lys Lys
            20                  25                  30

Ile Lys Glu Ile Lys Pro Asp Ile Val His Cys His Ser Ser Lys Ala
        35                  40                  45

Gly Val Ile Gly Arg Leu Ala Ala Lys Arg Arg Gly Val Lys Lys Ile
    50                  55                  60

Phe Tyr Thr Pro His Ala Tyr Ser Phe Leu Ala Pro Glu Phe Ser Gly
65                  70                  75                  80

Lys Lys Lys Phe Leu Phe Val Gln Ile Glu Lys Phe Leu Ser Arg Phe
                85                  90                  95

Ala Thr Thr Lys Ile Phe Cys Val Ser Ile Ala Glu Met Gln Ala Ala
            100                 105                 110

Leu Glu Val Asn Leu Asp Lys Thr Asp Lys Phe Gln Val Ile Tyr Asn
        115                 120                 125

Gly Leu Pro Glu Ile Asp Leu Pro Ser Lys Glu Thr Ile Arg Ala Gln
    130                 135                 140

Leu Gly Leu Glu Lys Ala Ala Val Val Ile Gly Asn Asn Ala Lys Met
145                 150                 155                 160

Ser Glu Gln Lys Asn Pro Met Phe Phe Met Glu Ile Ala Arg Lys Met
                165                 170                 175

Ile Arg Gln Asn Ala Asn Trp His Phe Val Trp Val Gly Asp Gly Gln
            180                 185                 190

Leu Met Pro Leu Phe Gln Ser Phe Ile Lys Gln Asn Gly Leu Glu Gly
        195                 200                 205

Asn Ile His Leu Leu Gly Glu Arg Pro Asp Ser Glu Ile Val Val Thr
    210                 215                 220

Ala Tyr Asp Ile Phe Leu Thr Thr Ser Gln Tyr Glu Gly Leu Pro Tyr
225                 230                 235                 240

Ala Pro Ile Glu Ala Met Arg Ala Gly Val Pro Ile Leu Ala Thr Lys
                245                 250                 255

Val Val Gly Asn Ser Glu Leu Val Ile Glu Gly Lys Asn Gly Tyr Leu
            260                 265                 270

Ile Asp Leu Glu Trp Ser Lys Ser Val Glu Glu Lys Leu Tyr Lys Ala
        275                 280                 285

Ala Lys Ile Asp Ala Gln Met Ile Lys Ala Asp Phe Arg Gln Arg Phe
    290                 295                 300

Ala Ile Asp Gln Ile Leu Lys Gln Ile Glu Thr Ile Tyr Leu Ala
305                 310                 315

(2) INFORMATIONS POUR LA SEQ ID NO: 8:
    (i) CARACTERISTIQUES DE LA SEQUENCE:
         (A) LONGUEUR: 384 acides aminés
         (B) TYPE: acide aminé
         (D) CONFIGURATION: linéaire
    (ii) TYPE DE MOLECULE: protéine
    (xi) DESCRIPTION DE LA SEQUENCE: SEQ ID NO: 8:
   Val Glu Cys Lys Leu Ser Asn Gly Glu Glu Asn Asn Met Lys Lys Ile
     1               5                  10                  15
   Ser Ile Leu His Phe Ser Gln Val Ser Gly Gly Gly Val Glu Lys Tyr
                20                  25                  30
   Ile Lys Leu Phe Leu Lys Tyr Ser Asp Val Thr Lys Phe Asn Asn Tyr
            35                  40                  45
   Leu Val Ala Pro Asn Leu Glu Asn Tyr Asp Glu Phe Asn Gly Tyr Leu
        50                  55                  60
   Lys Met Ser Val Asn Phe Asn Met Glu Gln Thr Phe Ser Pro Leu Lys
    65                  70                  75                  80
   Ile Phe Lys Asn Val Phe Phe Ile Arg Ser Val Leu Lys Lys Ile Asn
                    85                  90                  95
   Pro Asp Ile Val Tyr Leu His Ser Thr Phe Ala Gly Val Val Gly Arg
               100                 105                 110
   Ile Ala Ser Ile Gly Leu Pro Thr Lys Val Val Tyr Asn Pro His Gly
           115                 120                 125
   Trp Ser Phe Lys Met Asp Asn Ser Tyr Leu Lys Lys Leu Ile Phe Lys
       130                 135                 140
   Leu Ile Glu Phe Ser Leu Ser Phe Leu Thr Asp Lys Phe Ile Leu Ile
   145                 150                 155                 160
   Ser Glu Ser Glu Tyr Ile Leu Ala Asn His Ile Ser Phe Asn Lys Ser
                   165                 170                 175
   Lys Phe Ser Leu Ile Asn Asn Gly Val Glu Val Ile Thr Gly Asp Ser
               180                 185                 190
   Arg Asn Glu Ile Glu Glu Ile Phe Pro Asn Glu Asp Phe Ile Ile Gly
           195                 200                 205
   Met Val Gly Arg Leu Ser Pro Pro Lys Glu Phe Phe Phe Phe Ile Asp
       210                 215                 220
   Phe Ala Lys Lys Ile Leu Gln Ile Arg Asn Asp Thr Asn Phe Ile Ile
   225                 230                 235                 240
   Val Gly Asp Gly Glu Leu Arg Ser Glu Ile Glu Arg Met Ile Leu Asp
                   245                 250                 255
   Asn Gly Leu Gly Asp Lys Ile Tyr Ile Thr Gly Trp Val Asp Asn Pro
               260                 265                 270
   Arg Asn Tyr Ile Glu Lys Phe Asp Gln Ala Ile Leu Phe Ser Arg Trp
           275                 280                 285
   Glu Gly Leu Ser Leu Thr Ile Ala Glu Tyr Met Ser Gln Lys Lys Thr
       290                 295                 300
   Ile Leu Ala Thr Asn Ile Gly Gly Ile Asn Asp Leu Ile Thr Asp Gly
   305                 310                 315                 320
   Glu Thr Gly Met Leu Ile Glu Val Gly Asp Leu Asn Ser Ala Val Ser
                   325                 330                 335
   Lys Ser Phe Glu Leu Arg Asn Asn Lys Glu Val Ser Asn Gln Leu Ala
               340                 345                 350
   Asn Asn Ala Tyr Asn Lys Val Val Glu Gln Phe Ser Ile Glu Lys Gln
           355                 360                 365
   Met Ala Glu Ile Glu Ser Leu Phe Ile Glu Met Cys Asn Asn Glu Lys
       370                 375                 380

(2) INFORMATIONS POUR LA SEQ ID NO: 9:
    (i) CARACTERISTIQUES DE LA SEQUENCE:
        (A) LONGUEUR: 159 acides aminés
        (B) TYPE: acide aminé
        (D) CONFIGURATION: linéaire
    (ii) TYPE DE MOLECULE: protéine
    (xi) DESCRIPTION DE LA SEQUENCE: SEQ ID NO: 9:

Met Leu Ile Leu Lys Leu Lys Phe His Leu Lys Ser Leu Phe Leu Lys
1               5                   10                  15

Trp Ile Tyr Arg Leu Leu Tyr Leu Lys Lys Phe Gln Phe Gly Ala Arg
            20                  25                  30

Leu Thr Phe Arg Asp Gly Phe His Leu Leu Ile Glu Lys Ser Gly Lys
        35                  40                  45

Val Ile Ile Gly Asn His Val Phe Phe Asn Asn Phe Cys Ser Ile Asn
    50                  55                  60

Ala Met Leu Ser Val Thr Ile Gly Asp Asp Cys Ile Phe Gly Glu Asn
65                  70                  75                  80

Val Lys Ile Tyr Asp His Asn His Cys Tyr Gln Asn Lys Ser Gln Pro
                85                  90                  95

Ile Ser Lys Gln Gly Phe Ser Thr Ala Ala Ile Gln Ile Gly Arg Asn
            100                 105                 110

Cys Trp Ile Gly Ser Gln Val Thr Ile Leu Lys Gly Val Thr Ile Gly
        115                 120                 125

Asp Asn Ser Ile Ile Gly Ala Gly Val Val Val Tyr Gln Asp Val Pro
    130                 135                 140

Glu Asn Ser Ile Val Leu Ser Asn Gly Glu Ile Arg Lys Arg Gly
145                 150                 155

(2) INFORMATIONS POUR LA SEQ ID NO: 10:
    (i) CARACTERISTIQUES DE LA SEQUENCE:
        (A) LONGUEUR: 324 acides aminés
        (B) TYPE: acide aminé
        (D) CONFIGURATION: linéaire
    (ii) TYPE DE MOLECULE: protéine
    (xi) DESCRIPTION DE LA SEQUENCE: SEQ ID NO: 10:

Met Tyr Leu Lys Ser Leu Ile Ser Ile Val Ile Pro Val Tyr Asn Val
  1               5                  10                  15

Glu Lys Tyr Leu Glu Lys Cys Leu Gln Ser Val Gln Asn Gln Thr Tyr
            20                  25                  30

Asn Asn Phe Glu Val Ile Leu Val Asn Asp Gly Ser Thr Asp Ser Ser
        35                  40                  45

Leu Ser Ile Cys Glu Lys Phe Val Asn Gln Asp Lys Arg Phe Ser Val
    50                  55                  60

Phe Ser Lys Glu Asn Gly Gly Met Ser Ser Ala Arg Asn Phe Gly Ile
65                  70                  75                  80

Lys Lys Ala Lys Gly Ser Phe Ile Thr Phe Val Asp Ser Asp Asp Tyr
                85                  90                  95

Ile Val Lys Asp Tyr Leu Ser His Leu Val Ala Gly Ile Lys Ser Glu
            100                 105                 110

Thr Ser Ile Val Cys Ser Lys Phe Phe Leu Val Asp Glu Lys Gly Ser
        115                 120                 125

Leu Leu Thr Lys Lys Glu Ala Pro Lys Lys Lys Ser Glu Val Val Ser
    130                 135                 140

Ile Glu Glu Ser Ile Lys Ile Leu Leu Leu Gln Gln Asn Gly Tyr Asp
145                 150                 155                 160

Leu Ala Val Trp Gly Lys Leu Tyr Pro Val Ser Phe Phe Glu Thr Ile
                165                 170                 175

Ser Phe Pro Glu Gly Lys Leu Tyr Glu Asp Met Gly Thr Thr Tyr Lys
            180                 185                 190

Leu Leu Lys Leu Ala Ser Glu Val Val Phe Leu Asp Ala Tyr Asp Tyr
        195                 200                 205

Ala Tyr Val Gln Arg Pro Asn Ser Ile Met Asn Ser Ser Phe Asn Leu
    210                 215                 220

Lys Lys Leu Asp Ile Ile Glu Met Val His Glu Met Glu Asn Asp Ile
225                 230                 235                 240

Leu Ala Gln Phe Pro Asn Leu Ala Leu Tyr Val Lys Asn Arg Ala Phe
                245                 250                 255

Ala Ala Glu Val Lys Ile Phe Leu Glu Ile Pro Lys Glu Lys Glu Phe
            260                 265                 270

Glu Gln Ala Gln Lys Gln Leu Trp His Asp Ile Lys Lys Asn Arg Lys
        275                 280                 285

Ala Pro Phe Met Thr Lys Gly Ala Arg Leu Lys Asn Arg Leu Gly Ala
    290                 295                 300

Ser Leu Ser Phe Leu Gly Lys Ser Leu Phe Leu Thr Ile Gly Lys Gln
305                 310                 315                 320

Leu Val Asp Arg

(2) INFORMATIONS POUR LA SEQ ID NO: 11:
        (i) CARACTERISTIQUES DE LA SEQUENCE:
            (A) LONGUEUR: 360 acides aminés
            (B) TYPE: acide aminé
            (D) CONFIGURATION: linéaire
    (ii) TYPE DE MOLECULE: protéine
    (xi) DESCRIPTION DE LA SEQUENCE: SEQ ID NO: 11:

Met Val Ile Tyr Phe Leu Leu Phe Pro Met Ile Ala Met Ile Tyr Leu
  1               5                  10                  15

Met Thr Leu Leu Leu Arg Gln Lys Ala Gln Ile Gln Lys Thr Ile Phe
             20                  25                  30

Cys Val Leu Thr Phe Gly Thr Leu Gly Phe Ile Ser Ala Ser Arg Ala
         35                  40                  45

Ser Ser Val Gly Thr Asp Val Thr Leu Tyr Glu Asn Ile Phe Lys Ser
     50                  55                  60

Ile Asn Tyr Gly Ile Ser Ala Glu Asn Asn Trp Gly Tyr Val Ile Tyr
 65                  70                  75                  80

Asn Lys Leu Ile Gly Ser Val Phe Gly Tyr Thr Gly His Glu Ile Thr
                 85                  90                  95

Ala Ala Asn Ser Val Leu Ile Thr Ile Leu Ile Gly Ile Phe Ile Trp
            100                 105                 110

Lys Val Ala Glu His Tyr Phe Val Ala Thr Phe Leu Tyr Ile Ser Leu
        115                 120                 125

Phe Tyr Tyr Ala Thr Ser Phe Asn Ile Ser Arg Gln Phe Ile Ala Met
    130                 135                 140

Gly Leu Val Leu Val Ala Ile Ser Phe Ala Leu Asp Lys Lys Val Met
145                 150                 155                 160

Pro Trp Phe Ile Leu Thr Val Leu Ala Thr Leu Phe His Ala Thr Ala
                165                 170                 175

Ile Val Ala Phe Pro Val Tyr Trp Leu Thr Lys Val His Trp Asp Val
            180                 185                 190

Lys Lys Thr Leu Ser Ile Phe Pro Ile Thr Ile Phe Ala Ser Phe Ile
        195                 200                 205

Phe Asp Ala Ile Leu Asn Ile Phe Val Arg Phe Phe Pro His Tyr Glu
    210                 215                 220

Met Tyr Ile Thr Gly Thr Gln Phe Asn Ile Ser Asp Gln Gly Gln Gly
225                 230                 235                 240

Arg Val Val Leu Val Lys Ile Phe Ile Leu Leu Ile Leu Phe Thr Leu
                245                 250                 255

Phe Leu Phe Tyr Lys Lys Ser Tyr Ala Leu Ile Ser Glu Cys His Gln
            260                 265                 270

Ser Leu Ile Ala Leu Thr Thr Val Gly Leu Ser Ile Gly Ile Val Phe
        275                 280                 285

Tyr Asn Asn Ile Leu Leu Asn Arg Ile Glu Met Phe Tyr Ser Ile Leu
    290                 295                 300

Ser Ile Val Phe Ile Pro Ile Ala Ile Asp Tyr Ile Ser Leu Lys Phe
305                 310                 315                 320

Lys Gln Lys Asp Ala Val Arg Leu Met Leu Thr Ile Gly Ile Leu Leu
                325                 330                 335

Ile Thr Leu Val Pro Tyr Tyr Ile Gln Val Ser Gly Asn Tyr Ser Gly
            340                 345                 350

Ile Leu Pro Tyr Val Ile Gln Gln
        355                 360

(2) INFORMATIONS POUR LA SEQ ID NO: 12:
     (i) CARACTERISTIQUES DE LA SEQUENCE:
          (A) LONGUEUR: 316 acides aminés
          (B) TYPE: acide aminé
          (D) CONFIGURATION: linéaire
    (ii) TYPE DE MOLECULE: protéine
    (xi) DESCRIPTION DE LA SEQUENCE: SEQ ID NO: 12:

Met Glu Asp Arg Lys Lys Gln Val Ile Leu Ile Leu Ser His Arg Asn
  1               5                  10                  15

Thr Leu Ala Leu Lys Ser Thr Ile Glu Leu Leu Asp Ser Gln Tyr Phe
             20                  25                  30

Asp Phe Phe Leu His Ile Asp Lys Lys Ser Arg Ile Gln Asp Phe Phe
         35                  40                  45

Tyr Leu Lys Lys Ile Thr Lys Phe Ser Thr Ile His Phe Ser Glu Arg
     50                  55                  60

Lys Asn Val His Trp Gly Gly Phe Ser Met Val Glu Ala Met Phe Ala
 65                  70                  75                  80

Leu Leu Glu Cys Ala Arg Asp Thr Gly Glu Tyr Ser Tyr Phe His Phe
                 85                  90                  95

Leu Ser Gly Asp Asp Met Pro Ile Lys Asp Asn Glu Ile Val Phe Asn
            100                 105                 110

Phe Phe Glu Asn Ser Tyr Pro Lys Asn Phe Ile Asp Ile Leu Asp Phe
        115                 120                 125

Glu Asn Val Asn Lys Asn Ser Tyr Phe Tyr Glu Pro Pro Glu Met Ile
    130                 135                 140

Glu Glu Arg Val Lys Tyr Tyr Tyr Pro His Met Asp Ile Leu Asn Arg
145                 150                 155                 160

Lys Gly Thr Asn Phe Ile Gly Lys Lys Leu Ile Tyr Leu Gln Lys Leu
                165                 170                 175

Leu Lys Val Asn Arg Leu Lys Asn Arg Glu Ile Glu Ile Phe Lys Gly
            180                 185                 190

His Gln Trp Cys Ser Leu Thr Asn Gln Phe Val Asp Ile Leu Leu Asp
        195                 200                 205

Lys Glu Glu Arg Arg Val Gly Lys Ser Tyr Phe Ser Ser Ser Leu Ile
    210                 215                 220

Pro Asp Glu Cys Tyr Phe Gln Thr Phe Ala Met Ile Lys Lys Val Glu
225                 230                 235                 240

Ile Tyr Gln Gln Lys Asn Met Ser Ala Arg Leu Ile Asp Trp Thr Arg
                245                 250                 255

Gly Lys Pro Tyr Ile Trp Arg Gln Asp Asp Phe Phe Glu Ile Met Asn
            260                 265                 270

Asp Lys Asp Ser Met Phe Ser Arg Lys Phe Asp Glu Asn Val Asp Arg
        275                 280                 285

Lys Ile Ile Glu Glu Ile Tyr Ile Lys Ile Arg Gly Arg Ser Thr Asp
    290                 295                 300

Glu Ala Asn Lys Ile Lys Asp Lys Arg Phe Thr Lys
305                 310                 315

(2) INFORMATIONS POUR LA SEQ ID NO: 13:
    (i) CARACTERISTIQUES DE LA SEQUENCE:
        (A) LONGUEUR: 432 acides aminés
        (B) TYPE: acide aminé
        (D) CONFIGURATION: linéaire
    (ii) TYPE DE MOLECULE: protéine
    (xi) DESCRIPTION DE LA SEQUENCE: SEQ ID NO: 13:

Met Asn Lys Tyr Lys Lys Leu Leu Ser Asn Ser Leu Val Phe Thr Ile
  1               5                  10                  15

Gly Asn Leu Gly Ser Lys Leu Leu Val Phe Leu Leu Val Pro Leu Tyr
             20                  25                  30

Thr Tyr Ala Met Thr Pro Gln Glu Tyr Gly Met Ala Asp Leu Tyr Gln
         35                  40                  45

Thr Thr Ala Asn Leu Leu Leu Pro Leu Ile Thr Met Asn Val Phe Asp
     50                  55                  60

Ala Thr Leu Arg Phe Ala Met Glu Lys Ser Met Thr Lys Glu Ser Val
 65                  70                  75                  80

Leu Thr Asn Ser Leu Val Val Trp Cys Phe Ser Ala Val Phe Thr Cys
                 85                  90                  95

Leu Gly Ala Cys Ile Ile Tyr Ala Leu Asn Leu Ser Asn Lys Trp Tyr
            100                 105                 110

Leu Ala Leu Leu Leu Thr Phe Asn Leu Phe Gln Gly Gly Gln Ser Ile
        115                 120                 125

Leu Ser Gln Tyr Ala Arg Gly Ile Gly Lys Ser Lys Ile Phe Ala Ala
    130                 135                 140

Gly Gly Val Ile Leu Thr Phe Leu Thr Gly Ala Leu Asn Ile Leu Phe
145                 150                 155                 160

Leu Val Tyr Leu Pro Leu Gly Ile Thr Gly Tyr Leu Met Ser Leu Val
                165                 170                 175

Leu Ala Asn Val Gly Thr Ile Leu Phe Phe Ala Gly Thr Leu Ser Ile
            180                 185                 190

Trp Lys Glu Ile Ser Phe Lys Ile Ile Asp Lys Lys Leu Ile Trp Gln
        195                 200                 205

Met Leu Tyr Tyr Ala Leu Pro Leu Ile Pro Ser Ser Ile Leu Trp Trp
    210                 215                 220

Leu Leu Asn Ala Ser Ser Arg Tyr Phe Val Leu Phe Phe Leu Gly Ala
225                 230                 235                 240

Gly Ala Asn Gly Leu Leu Ala Val Ala Thr Lys Ile Pro Ser Ile Ile
                245                 250                 255

Ser Ile Phe Asn Thr Ile Phe Thr Gln Ala Trp Gln Ile Ser Ala Ile
            260                 265                 270

Glu Glu Tyr Asp Ser His Gln Lys Ser Lys Tyr Tyr Ser Asp Val Phe
        275                 280                 285

His Tyr Leu Ala Thr Phe Leu Leu Leu Gly Thr Ser Ala Phe Met Ile
    290                 295                 300

Val Leu Lys Pro Ile Val Glu Lys Val Val Ser Ser Asp Tyr Ala Ser
305                 310                 315                 320

Ser Trp Gln Tyr Val Pro Phe Phe Met Leu Ser Met Leu Phe Ser Ser
                325                 330                 335

Phe Ser Asp Phe Phe Gly Thr Asn Tyr Ile Ala Ala Lys Gln Thr Lys
            340                 345                 350

Gly Val Phe Met Thr Ser Ile Tyr Gly Thr Ile Val Cys Val Leu Leu
        355                 360                 365

Gln Val Val Leu Leu Pro Ile Ile Gly Leu Asp Gly Ala Gly Leu Ser
    370                 375                 380

Ala Met Leu Gly Phe Leu Thr Thr Phe Leu Leu Arg Val Lys Asp Thr
385                 390                 395                 400

Gln Lys Phe Val Val Ile Gln Ile Lys Trp Arg Ile Phe Ile Ser Asn
                405                 410                 415

Leu Leu Ile Val Phe Gly Thr Asn Phe Met Phe Val Leu Ser Thr Glu
            420                 425                 430

(2) INFORMATIONS POUR LA SEQ ID NO: 14:
    (i) CARACTERISTIQUES DE LA SEQUENCE:
        (A) LONGUEUR: 307 acides aminés
        (B) TYPE: acide aminé
        (C) NOMBRE DE BRINS: simple
        (D) CONFIGURATION: lin,aire
    (ii) TYPE DE MOLECULE: peptide
    (xi) DESCRIPTION DE LA SEQUENCE: SEQ ID NO: 14:

Met Lys Gln Ile Lys Ser Lys Ile Arg Asp Leu Gln Asn Asn Phe Thr
1               5                   10                  15

Tyr Val Phe Gly Lys Lys Thr Phe Leu Gly Arg Gly Glu Ala Ile Ile
            20                  25                  30

Ile Asp Glu Pro Glu His Gly Asn Leu Gly Asp Gln Ala Ile Ala Phe
        35                  40                  45

Ala Glu Asn Gln Phe Leu Val Asn His Val Ser Val Arg Asp Val Glu
    50                  55                  60

His Leu Ile Glu Ser Lys Thr Ile Ser Glu Ile Lys Ser Ile Lys Lys
65                  70                  75                  80

Asn Ile Gly Lys Lys Glu Leu Val Phe Phe His Gly Gly Gly Asn Phe
                85                  90                  95

Gly Thr Leu Tyr Leu Lys Tyr Glu Arg Ile Arg Arg Leu Ala Val Ser
            100                 105                 110

Lys Leu Pro Phe Asn Lys Met Ile Leu Phe Pro Gln Ser Ile Ser Phe
        115                 120                 125

Glu Asp Ser Arg Phe Gly Gln Lys Gln Leu Asn Lys Ser Lys Lys Ile
    130                 135                 140

Tyr Ser Gln Asn Thr Asn Phe Ile Leu Thr Ala Arg Glu Pro Lys Ser
145                 150                 155                 160

Tyr Gly Leu Met Lys Lys Cys Phe Pro Tyr Asn Lys Val Ile Leu Thr
                165                 170                 175

Pro Asp Ile Val Leu Ser Phe Lys Phe Glu Val Thr Ile Ser Asp Thr
            180                 185                 190

His Ile Gly Lys Glu Lys Asp Ser Val Ile Thr Tyr Glu Asn Arg Gln
        195                 200                 205

His Tyr Leu Glu Ile Lys Trp Asp Glu Ile Ala Gln His Glu Val Ala
    210                 215                 220

Leu Thr Asp Arg Leu His Gly Met Ile Phe Ser Tyr Ile Thr Gly Thr
225                 230                 235                 240

Pro Cys Val Val Leu Ala Asn Asn Asn His Lys Ile Glu Gly Thr Tyr
                245                 250                 255

Lys His Trp Leu Asn Glu Val Asn Tyr Ile Arg Phe Ile Glu Asn Pro
            260                 265                 270

Thr Val Glu Asn Ile Leu Asp Ala Ile Asn Asp Leu Lys Gln Ile Glu
        275                 280                 285

Pro His Tyr Ile Asp Leu Ser Asp Lys Phe Gln Pro Leu Ile Asp Ala
    290                 295                 300

Ile Lys Gly
305

(2) INFORMATIONS POUR LA SEQ ID NO: 15:
    (i) CARACTERISTIQUES DE LA SEQUENCE:
        (A) LONGUEUR: 32 paires de bases
        (B) TYPE: nucléotide
        (C) NOMBRE DE BRINS: simple
        (D) CONFIGURATION: linéaire
    (ii) TYPE DE MOLECULE: Autre acide nucl,ique
        (A) DESCRIPTION: /desc = "oligonucléotide"
    (xi) DESCRIPTION DE LA SEQUENCE: SEQ ID NO: 15:

GTTGCGGCCG CGATAAAGTG TGATAAGTCC AG                                32

(2) INFORMATIONS POUR LA SEQ ID NO: 16:
    (i) CARACTERISTIQUES DE LA SEQUENCE:
        (A) LONGUEUR: 30 paires de bases
        (B) TYPE: nucléotide
        (C) NOMBRE DE BRINS: simple
        (D) CONFIGURATION: linéaire
    (ii) TYPE DE MOLECULE: Autre acide nucléique
        (A) DESCRIPTION: /desc = "oligonucléotide"

(xi) DESCRIPTION DE LA SEQUENCE: SEQ ID NO: 16:

ATAGCGGCCG CTTAGCTCAT GTTGATGCGG 30

(2) INFORMATIONS POUR LA SEQ ID NO: 17:
(i) CARACTERISTIQUES DE LA SEQUENCE:
(A) LONGUEUR: 31 paires de bases
(B) TYPE: nucléotide
(C) NOMBRE DE BRINS: simple
(D) CONFIGURATION: linéaire
(ii) TYPE DE MOLECULE: Autre acide nucléique
(A) DESCRIPTION: /desc = "oligonucléotide"
(xi) DESCRIPTION DE LA SEQUENCE: SEQ ID NO: 17:

CCTGCGGCCG CGCTTCCTAA TTCTGTAATC G 31

(2) INFORMATIONS POUR LA SEQ ID NO: 18:
(i) CARACTERISTIQUES DE LA SEQUENCE:
(A) LONGUEUR: 31 paires de bases
(B) TYPE: nucléotide
(C) NOMBRE DE BRINS: simple
(D) CONFIGURATION: linéaire
(ii) TYPE DE MOLECULE: Autre acide nucléique
(A) DESCRIPTION: /desc = "oligonucléotide"
(xi) DESCRIPTION DE LA SEQUENCE: SEQ ID NO: 18:

TACGCGGCCG CACATAGAAT AAGGCTTTAC G 31

(2) INFORMATIONS POUR LA SEQ ID NO: 19:
(i) CARACTERISTIQUES DE LA SEQUENCE:
(A) LONGUEUR: 31 paires de bases
(B) TYPE: nucléotide
(C) NOMBRE DE BRINS: simple
(D) CONFIGURATION: linéaire
(ii) TYPE DE MOLECULE: Autre acide nucléique
(A) DESCRIPTION: /desc = "oligonucléotide"
(xi) DESCRIPTION DE LA SEQUENCE: SEQ ID NO: 19:

CTGGCGGCCG CTACTTCACG TTTCTTTGCA T 31